(19) Europäisches Patentamt
European Patent Office
Office européen des brevets

(11) EP 0 802 197 A1

(12) **EUROPÄISCHE PATENTANMELDUNG**

(43) Veröffentlichungstag: **22.10.1997 Patentblatt 1997/43**

(21) Anmeldenummer: **97105595.9**

(22) Anmeldetag: **04.04.1997**

(51) Int. Cl.$^6$: **C07D 471/04**, A61K 31/44 // (C07D471/04, 221:00, 209:00)

(84) Benannte Vertragsstaaten: **AT BE CH DE DK ES FI FR GB GR IE IT LI LU MC NL PT SE**

(30) Priorität: **17.04.1996 DE 19615119**

(71) Anmelder: **BAYER AG**
**51368 Leverkusen (DE)**

(72) Erfinder:
- **Eckenberg, Peter, Dr.**
  **40699 Erkrath (DE)**
- **Müller, Ulrich, Dr.**
  **42111 Wuppertal (DE)**
- **Grützmann, Rudi, Dr.**
  **42657 Solingen (DE)**
- **Bischoff, Hilmar, Dr.**
  **42113 Wuppertal (DE)**
- **Denzer, Dirk, Dr.**
  **42115 Wuppertal (DE)**
- **Nielsch, Ulrich, Dr.**
  **42113 Wuppertal (DE)**

(54) **Trizyklisch-Heterocyclyl substituierte Phenylessigsäureamid Derivate, deren Herstellung und deren Verwendung als Angiostensin-II-Antagonisten**

(57) Arylessigsäureamide der allgemeinen Formel (I)

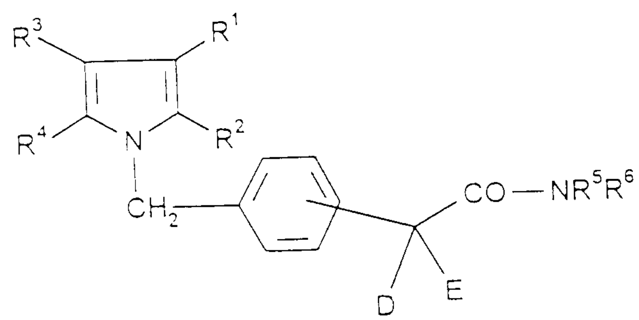

(I),

in welcher

$R^1$ und $R^2$ unter Einbezug der sie verbindenden Doppelbindung gemeinsam einen gegebenenfalls substituierten Phenyl- oder Pyridylring oder einen Ring der Formel

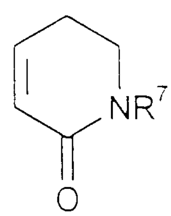

bilden,

$R^3$ und $R^4$ unter Einbezug der sie verbindenden Doppelbindung gemeinsam einen gegebenenfalls substituierten

EP 0 802 197 A1

Phenylring oder 4- bis 8-gliedrigen Cycloalken- oder Oxocycloalken-Rest bilden,

D und E gleich oder verschieden sind und
für Wasserstoff, Cycloalkyl mit 3 bis 8 Kohlenstoffatomen oder geradkettiges oder verzweigtes Alkyl mit bis zu 10 Kohlenstoffatomen stehen, das gegebenenfalls durch Cycloalkyl mit 3 bis 6 Kohlenstoffatomen substituiert ist, oder für Phenyl stehen, das gegebenenfalls durch Halogen oder Trifluormethyl substituiert ist, oder

D und E gemeinsam unter Einbezug der CH-Gruppe einen 4- bis 8- gliedrigen Carbocyclus bilden,

$R^5$ für Wasserstoff, geradkettiges oder verzweigtes Alkyl mit bis zu 12 Kohlenstoffatomen oder für Cycloalkyl mit 3 bis 8 Kohlenstoffatomen steht,

$R^6$ für Cycloalkyl mit 3 bis 8 Kohlenstoffatomen oder Phenyl steht, oder für gegebenenfalls substituiertes geradkettiges oder verzweigtes Alkyl mit bis zu 9 Kohlenstoffatomen steht, oder

$R^6$ für einen Rest der Formel $-(CH_2)_n-R^8$ steht, oder
für einen Rest der Formel

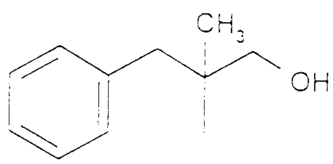

oder

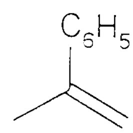

steht,
oder

$R^5$ und $R^6$ gemeinsam mit dem Stickstoffatom einen heterocyclischen Rest der Formel $-(CH_2)_2-O-(CH_2)_2$, $-CH_2-(CH_2)_p-CH_2-$,

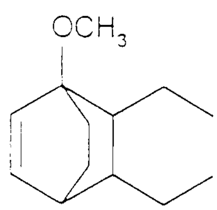

oder

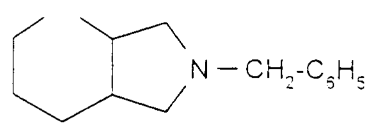

bilden; gegebenenfalls in einer isomeren Form und deren Salze.

Die erfindungsgemäßen Verbindungen zeichnen sich durch eine Senkung der ApoB-100-assoziierten Lipoproteine (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

## Beschreibung

Die vorliegende Erfindung betrifft neue Arylessigsäureamide, Verfahren zu ihrer Herstellung und ihre Verwendung als Arzneimittel, insbesondere als antiatherosklerotische Arzneimittel.

Es ist bekannt, daß erhöhte Blutspiegel von Triglyzeriden (Hypertriglyzeridämie) und Cholesterin (Hypercholesterinämie) mit der Genese von atherosklerotischen Gefäßwand-Veränderungen und koronaren Herzkrankheiten assoziiert sind.

Ein deutlich erhöhtes Risiko für die Entwicklung koronarer Herzerkrankungen liegt darüber hinaus vor, wenn diese beiden Risikofaktoren kombiniert auftreten, was wiederum mit einer Überproduktion an Apoliprotein B-100 einhergeht. Es ist daher nach wie vor ein starkes Bedürfnis, wirksame Arzneimittel zur Bekämpfung der Atherosklerose sowie koronarer Herzkrankheiten zur Verfügung zu stellen.

Die vorliegende Erfindung betrifft neue Arylessigsäureamide der allgemeinen Formel (I)

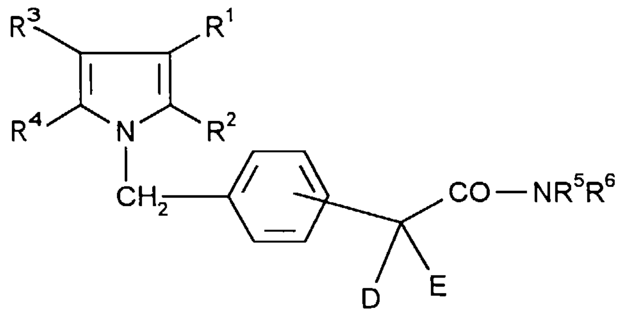

(I),

in welcher

$R^1$ und $R^2$ unter Einbezug der sie verbindenden Doppelbindung gemeinsam einen Phenyl- oder Pyridylring oder einen Ring der Formel

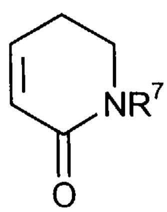

bilden,
worin

$R^7$ Wasserstoff oder geradkettiges oder verzweigtes Alkyl mit bis zu 4 Kohlenstoffatomen bedeutet,

$R^3$ und $R^4$ unter Einbezug der sie verbindenden Doppelbindung gemeinsam einen Phenylring oder einen 4- bis 8-gliedrigen Cycloalken- oder Oxocycloalken-Rest bilden,
wobei alle unter $R^1$/$R^2$ und $R^3$/$R^4$ aufgeführten Ringsysteme gegebenenfalls bis zu 3-fach gleich oder verschieden durch Halogen, Trifluormethyl, Carboxy, Hydroxy, durch geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert sein kann,

D und E gleich oder verschieden sind und
für Wasserstoff, Cycloalkyl mit 3 bis 8 Kohlenstoffatomen oder geradkettiges oder verzweigtes Alkyl mit bis zu 10 Kohlenstoffatomen stehen, das gegebenenfalls durch Cycloalkyl mit 3 bis 6 Kohlenstoffatomen substituiert ist, oder
für Phenyl stehen, das gegebenenfalls durch Halogen oder Trifluormethyl substituiert ist,
oder

D und E gemeinsam unter Einbezug der CH-Gruppe einen 4- bis 8-gliedrigen Carbocyclus bilden,

$R^5$ für Wasserstoff, geradkettiges oder verzweigtes Alkyl mit bis zu 12 Kohlentsoffatomen oder für Cycloalkyl mit 3 bis 8 Kohlenstoffatomen steht,

$R^6$ für Cycloalkyl mit 3 bis 8 Kohlenstoffatomen oder Phenyl steht, oder für geradkettiges oder verzweigtes Alkyl mit bis zu 9 Kohlenstoffatomen steht, das gegebenenfalls durch Hydroxy, Naphthyl, Trifluormethyl oder durch einen Rest der Formel

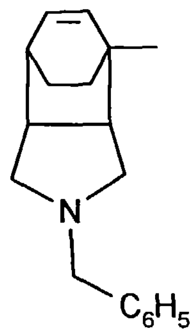

substituiert ist,
worin

a eine Zahl 1 oder 2 bedeutet
oder

$R^6$ für einen Rest der Formel $-(CH_2)_n-R^8$ steht,
worin

n eine Zahl 2, 3, 4 oder 5 bedeutet,

$R^8$ Naphthyl oder Phenyl bedeutet, die gegebenenfalls durch Carboxyl, Trifluormethyl, Halogen, Hydroxy, Trifluormethoxy oder durch geradkettiges oder verzweigtes Alkyl, Acyl, Alkoxy oder Alkoxycarbonyl mit jeweils bis zu 6 Kohlenstoffatomen substituiert sind,
oder
für einen Rest der Formel

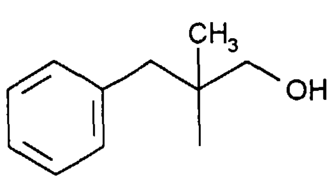 oder 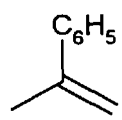 steht,

oder

$R^5$ und $R^6$ gemeinsam mit dem Stickstoffatom einen heterocyclischen Rest der Formel $-(CH_2)_2O-(CH_2)_2$, $-CH_2-(CH_2)_p-CH_2-$,

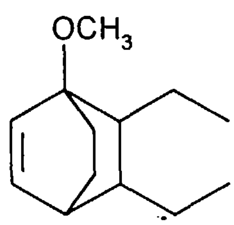 oder

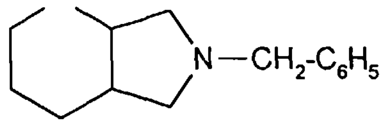

bilden
worin

p eine Zahl 2, 3, 4, 5, 6, 7, 8 oder 9 bedeutet,

gegebenenfalls in einer isomeren Form und deren Salze.

Die erfindungsgemäßen neuen Arylessigsäureamide können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di-bzw. Triethylamin, Di- bzw. Triethanolamin, Dicyclohexylamin, Dimethylaminoethanol, Arginin, Lysin, Ethylendiamin oder 2-Phenylethylamin.

Der Cycloalken-Rest ($R^3/R^4$) steht unter Einbezug der Doppelbindung des Grundgerüstes im Rahmen der Erfindung im allgemeinen für einen 4- bis 8-gliedrigen, vorzugsweise 5- bis 8-gliedrigen Kohlenwasserstoffrest wie beispielsweise einen Cyclobuten-, Cyclopenten-, Cyclohexen-, Cyclohepten- oder Cycloocten-Rest. Bevorzugt sind der Cyclopenten-, Cyclohexen-, Cycloocten- und Cyclohepten-Rest.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweiligen Mischungen. Diese Mischungen der Enantiomeren und Diastereomeren lassen sich in bekannter Weise in die stereoisomer einheitlichen Bestandteile trennen.

Bevorzugt sind Verbindungen der allgemeinen Formel (I),
in welcher

$R^1$ und $R^2$ unter Einbezug der sie verbindenden Doppelbindung gemeinsam einen Phenyl- oder Pyridylring oder einen Ring der Formel

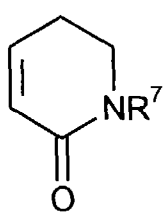

bilden,
worin

$R^7$ Wasserstoff oder geradkettiges oder verzweigtes Alkyl mit bis zu 3 Kohlenstoffatomen bedeutet,

$R^3$ und $R^4$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,

wobei alle unter $R^1/R^2$ und $R^3/R^4$ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 4 Kohlenstoffatomen oder durch geradkettiges

oder verzweigtes Alkyl mit bis zu 4 Kohlenstoffatomen substituiert sind, das seinerseits durch Hydroxy oder durch geradkettiges oder verzweigtes Alkoxy mit bis zu 3 Kohlenstoffatomen substituiert sein kann,

D und E gleich oder verschieden sind und für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 8 Kohlenstoffatomen stehen, das gegebenenfalls durch Cyclopropyl, Cylcopentyl oder Cyclohexyl substituiert ist, oder für Phenyl stehen, das gegebenenfalls durch Fluor, Chlor oder Brom substituiert ist,

oder

D und E gemeinsam unter Einbezug der CH-Gruppe einen Cyclobutyl-, Cyclopentyl-, Cyclohexyl-, Cycloheptyl- oder Cyclooctylring bilden,

$R^5$ für Wasserstoff geradkettiges oder verzweigtes Alkyl mit bis zu 10 Kohlenstoffatomen, Cyclopropyl, Cyclobutyl, Cyclopentyl, Cyclohexyl, Cycloheptyl oder Cyclooctyl steht,

$R^6$ für Cyclopropyl, Cyclobutyl, Cyclopentyl, Cyclohexyl, Cycloheptyl, Cyclooctyl oder Phenyl steht, oder für geradkettiges oder verzweigtes Alkyl mit bis zu 7 Kohlenstoffatomen steht, das gegebenenfalls durch Hydroxy, Naphthyl, Trifluormethyl oder durch einen Rest der Formel

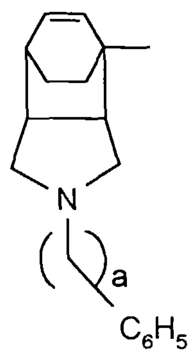

substituiert ist,

worin

a eine Zahl 1 oder 2 bedeutet

oder

$R^6$ für einen Rest der Formel $-(CH_2)_n-R^8$ steht,

worin

n eine Zahl 2, 3 oder 4 bedeutet,

$R^8$ Naphthyl oder Phenyl bedeutet, die gegebenenfalls durch Trifluormethyl, Fluor, Chlor, Brom, Hydroxy, Trifluormethoxy oder durch geradkettiges oder verzweigtes Alkyl, Alkoxy oder Alkoxycarbonyl mit jeweils bis zu 5 Kohlenstoffatomen substituiert sind,

oder

für einen Rest der Formel

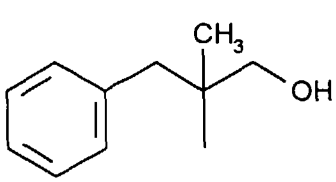 oder 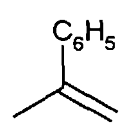 steht,

oder

$R^5$ und $R^6$ gemeinsam mit dem Stickstoffatom einen heterocyclischen Rest der Formel $-(CH_2)_2-O(CH_2)_2$, $-CH_2-(CH_2)_p-CH_2-$,

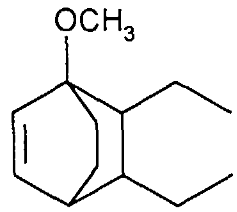

oder

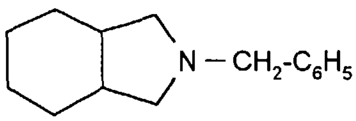

bilden
worin

p eine Zahl 2, 3, 4, 5, 6, 7 oder 8 bedeutet,

gegebenenfalls in einer isomeren Form und deren Salze.

Besonders bevorzugt sind Verbindungen der allgemeinen Formel (I), in welcher

$R^1$ und $R^2$ unter Einbezug der sie verbindenden Doppelbindung gemeinsam einen Phenyl- oder Pyridylring oder einen Ring der Formel

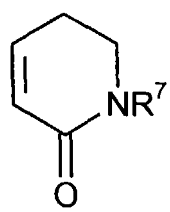

bilden,
worin

$R^7$ Wasserstoff oder Methyl bedeutet,

$R^3$ und $R^4$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten- oder Oxocycloocten-Rest bilden,
wobei alle unter $R^1$/$R^2$ und $R^3$/$R^4$ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 3 Kohlenstoffatomen oder durch geradkettiges oder verzweigtes Alkyl mit bis zu 3 Kohlenstoffatomen substituiert sind, das seinerseits durch Hydroxy, Methoxy oder Ethoxy substituiert sein kann,

D und E gleich oder verschieden sind und
für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 6 Kohlenstoffatomen stehen, das gegebenenfalls durch Cyclopentyl oder Cyclohexyl substituiert ist, oder
für Phenyl stehen, das gegebenenfalls durch Fluor, Chlor oder Brom substituiert ist,
oder

D und E gemeinsam unter Einbezug der CH-Gruppe einen Cyclopentyl-, Cyclohexyl- oder Cycloheptylring bilden,

$R^5$ für Wasserstoff, geradkettiges oder verzweigtes Alkyl mit bis zu 8 Kohlenstoffatomen, Cyclopentyl, Cyclohexyl oder Cycloheptyl steht,

$R^6$ für Cylcopentyl, Cyclooctyl oder Phenyl steht, oder

für geradkettiges oder verzweigtes Alkyl mit bis zu 6 Kohlenstoffatomen steht, das gegebenenfalls durch Hydroxy, Naphthyl, Trifluormethyl oder durch einen Rest der Formel

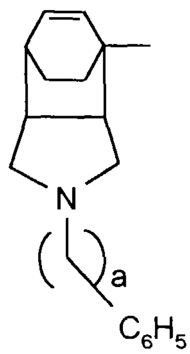

substituiert ist,

worin

a eine Zahl 1 oder 2 bedeutet

oder

$R^6$ für einen Rest der Formel $-(CH_2)_n-R^8$ steht,

worin

n eine Zahl 2 oder 3 bedeutet,

$R^8$ Naphthyl oder Phenyl bedeutet, die gegebenenfalls durch Trifluormethyl, Fluor, Chlor, Hydroxy, Trifluormethoxy oder durch geradkettiges oder verzweigtes Alkyl, Alkoxy oder Alkoxycarbonyl mit jeweils bis zu 3 Kohlenstoffatomen substituiert sind,

oder

für einen Rest der Formel

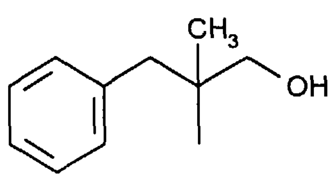 oder 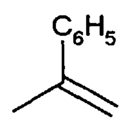 steht,

oder

$R^5$ und $R^6$ gemeinsam mit dem Stickstoffatom einen heterocyclischen Rest der Formel $-(CH_2)_2-O-(CH_2)_2$, $-CH_2-(CH_2)_p-CH_2-$,

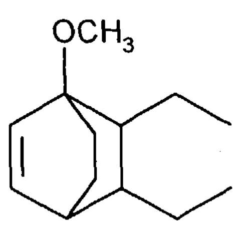 oder

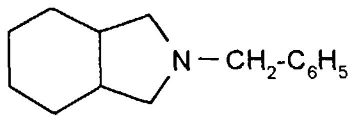

bilden
worin

p eine Zahl 2, 3, 4, 5, 6 oder 7 bedeutet,

gegebenenfalls in einer isomeren Form und deren Salze.

Außerdem wurde ein Verfahren gefunden, dadurch gekennzeichnet, daß man ausgehend von den (racemischen oder enantiomerenreinen) Carbonsäuren der allgemeinen Formel (II)

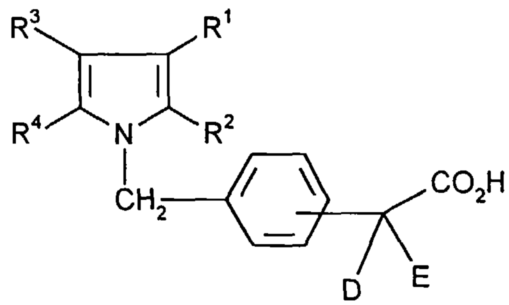

(II)

in welcher

D, E, $R^1$, $R^2$, $R^3$ und $R^4$ die angegebene Bedeutung haben,

zunächst die entsprechenden (racemischen oder enantiomerenreinen) Säurechloride der allgemeinen Formel (III)

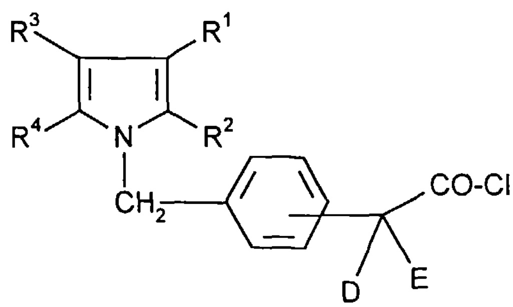

(III)

in welcher

D, E, $R^1$, $R^2$, $R^3$ und $R^4$ die angegebene Bedeutung haben,

herstellt
und abschließend mit Aminen der allgemeinen Formel (IV)

$$HNR^5R^6 \quad (IV)$$

in welcher

$R^5$ und $R^6$ die angegebene Bedeutung haben,

in inerten Lösemitteln, gegebenenfalls in Anwesenheit von Basen und/oder Hilfsmitteln umsetzt.

Das erfindungsgemäße Verfahren kann durch folgendes Formelschema beispielhaft erläutert werden:

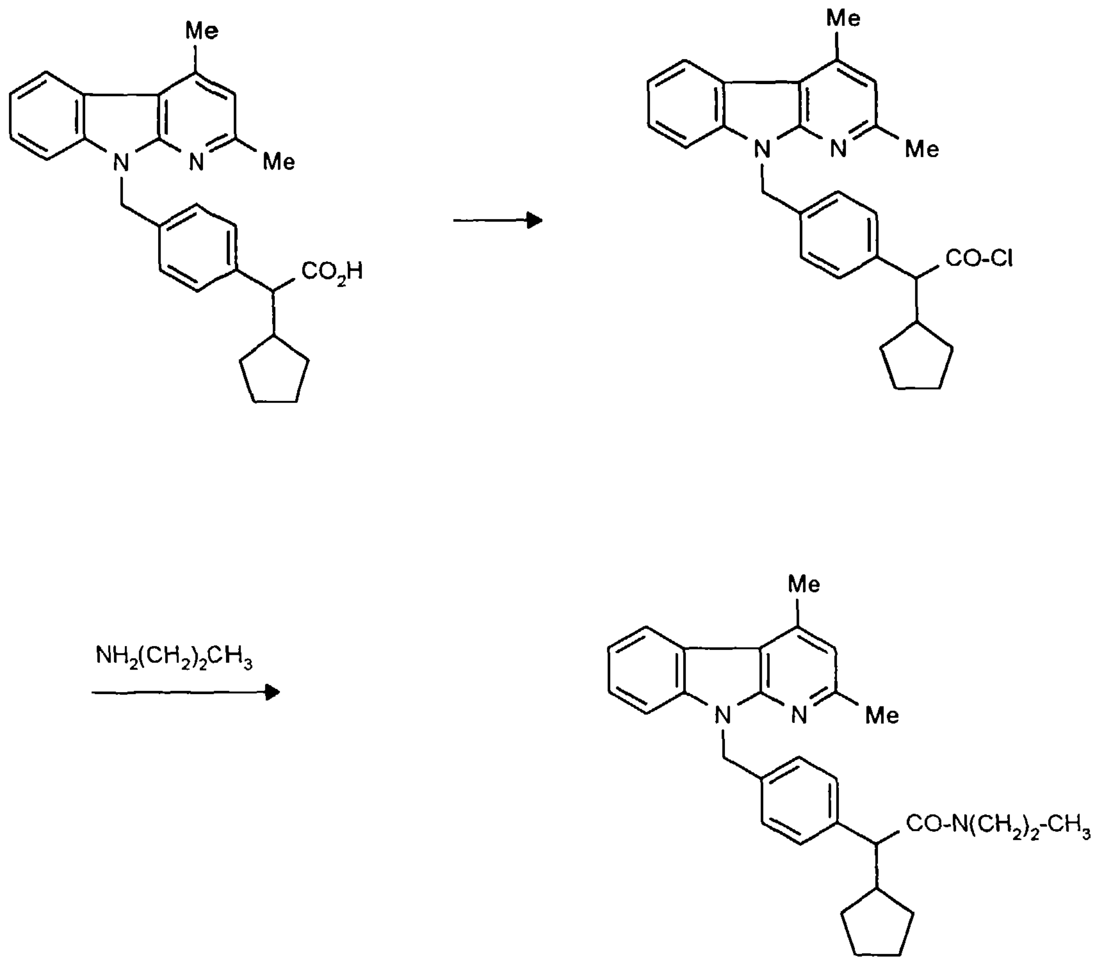


Als Lösemittel eignen sich hierbei inerte organische Lösemittel, die sich unter den Reaktionsbedingungen nicht verändern. Hierzu gehören Ether, wie Diethylether oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwasserstoffe wie Benzol, Xylol, Toluol, Hexan, Cyclohexan, oder Erdölfraktionen, Nitromethan, Dimethylformamid, Acetonitril oder Hexamethylphosphorsäuretriamid.

Ebenso ist es möglich, Gemische der Lösemittel einzusetzen. Besonders bevorzugt sind Dichlormethan, Tetrahydrofuran und Dimethylformamid.

Als Basen für das erfindungsgemäße Verfahren können im allgemeinen organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin eingesetzt werden. Es ist auch möglich als Basen Alkalimetalle wie Natrium und deren Hydride wie Natriumhydrid einzusetzen. Bevorzugt sind Pyridin und Triethylamin.

Die Base wird in einer Menge von 1 mol bis 5 mol, bevorzugt von 1 mol bis 3 mol, bezogen auf 1 mol der Verbindung der allgemeinen Formel (III) eingesetzt.

Die Reaktion wird im allgemeinen in einem Temperaturbereich von 0°C bis 150°C, bevorzugt von 0°C bis +25°C durchgeführt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Variation von funktionellen Gruppen wie beispielsweise Hydrolyse, Veresterung und Reduktion, sowie die Isomerentrennung und Salzbildung erfolgt nach üblichen Methoden.

Die racemischen Carbonsäuren der allgemeinen Formel (II) sind neu und können hergestellt werden, indem man zunächst durch Umsetzung von Verbindungen der allgemeinen Formel (V)

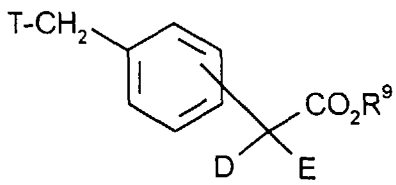

(V)

in welcher

| | |
|---|---|
| D und E | die angegebene Bedeutung haben, |
| T | für eine typische Abgangsgruppe wie beispielsweise Chlor, Brom, Jod, Tosylat oder Mesylat, vorzugsweise für Brom steht, und |
| $R^9$ | für $(C_1\text{-}C_4)$-Alkyl steht, mit Verbindungen der allgemeinen Formel (VI) |

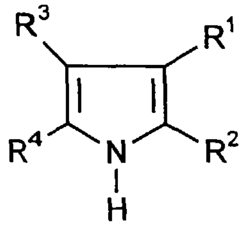

(VI)

in welcher

| | |
|---|---|
| $R^1$, $R^2$, $R^3$ und $R^4$ | die angegebene Bedeutung haben, die Verbindungen der allgemeinen Formel (VII) |

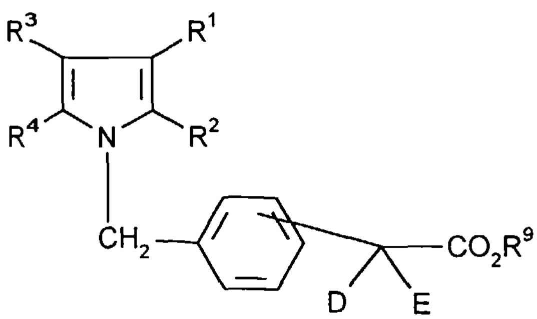

(VII)

in welcher

| | |
|---|---|
| D, E, $R^1$, $R^2$, $R^3$, $R^4$ und $R^9$ | die angegebene Bedeutung haben, |

in inerten Lösemitteln, gegebenenfalls in Anwesenheit von Basen herstellt und anschließend die Ester nach üblichen Methoden verseift.

Die enantiomerenreinen Säuren, d.h. Verbindungen der Formel (II), bei denen D und E verschieden sein müssen, erhält man darüber hinaus, indem man zunächst ausgehend von den D- oder L-Menthyl-Estern der allgemeinen Formel (VIII)

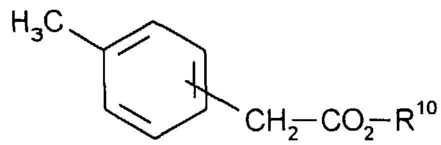

(VIII)

in welcher

$R^{10}$ für D- oder L-Menthyl steht,

durch Umsetzung mit Verbindungen der allgemeinen Formeln (IXa) und (IXb)

D-Z (IXa)

E-Z (IXb)

in welcher

D bzw. E verschieden sind und ansonsten die angegebene Bedeutung haben, und

Z für Halogen, vorzugsweise Brom steht,

die enantiomerenreinen Menthyl-Ester der allgemeinen Formeln (Xa) und (Xb)

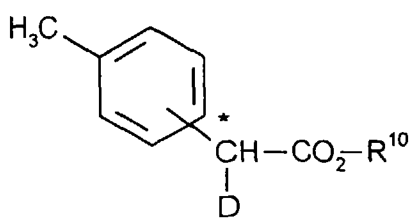

(Xa)

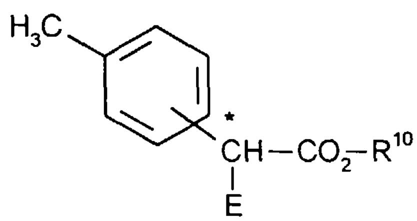

(Xb)

in welcher

D, E und $R^{10}$ die angegebene Bedeutung haben,

herstellt,
diese in einem nächsten Schritt durch eine Halogenierung in die Verbindungen der allgemeinen Formeln (XIa) und (XIb)

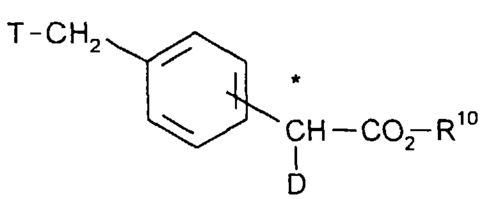

(XIa)

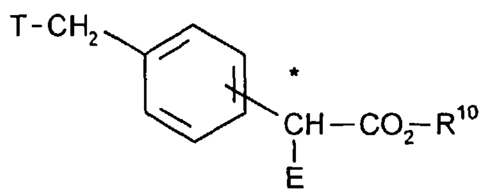

(XIb)

in welcher

D, E, T und $R^{10}$ die angegebene Bedeutung haben

überführt,
aus diesen anschließend durch Umsetzung mit den Verbindungen der allgemeinen Formel (V) die enantiomerenreinen Verbindungen der allgemeinen Formeln (XIIa) und (XIIb)

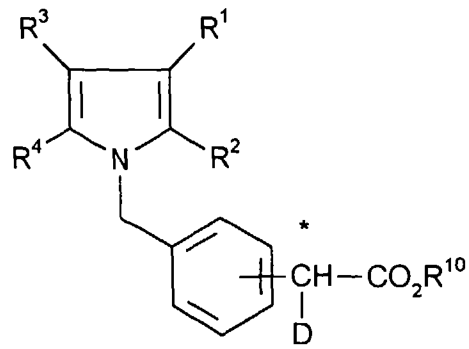

(XIIa)

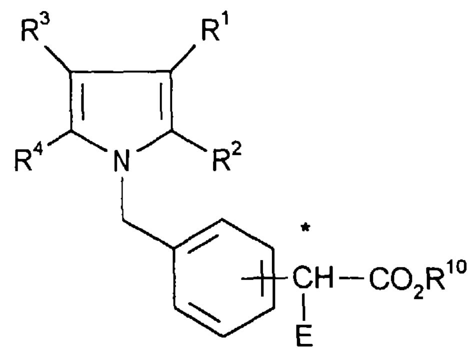

(XIIb)

in welcher

D, E, $R^1$, $R^2$, $R^3$, $R^4$ und $R^{10}$ die angegebene Bedeutung haben,

herstellt,
und diese dann durch Hydrolyse in die enantiomerenreinen Säuren der allgemeinen Formel (II) überführt.

Außerdem können die enantiomerenreinen Säuren der Formel (II) hergestellt werden, indem man zunächst racemische Carbonsäuren der allgemeinen Formel (XIII)

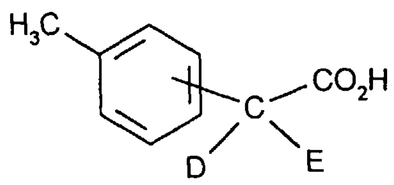

(XIII)

in welcher

D und E die oben angegebene Bedeutung haben,

durch Umsetzung mit (R)- oder (S)-Phenylethylamin in inerten Lösemitteln und anschließender Kristallisation der Phenethylammoniumsalze und anschließende Hydrolyse der Salze in die enantiomerenreinen Verbindungen der allgemeinen Formel (XIVa,b)

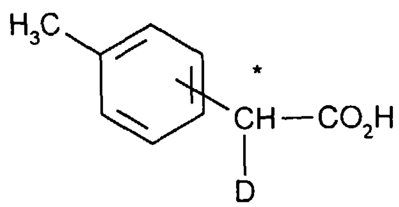

(XIVa)

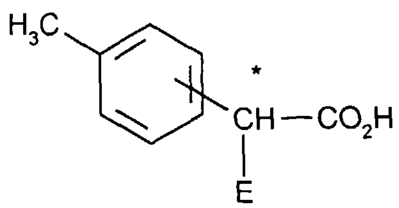

(XIVb)

in welcher

D und E die oben angegebene Bedeutung haben,

überführt,
in einem weiteren Schritt mit Isobuten, in inerten Lösemitteln und in Anwesenheit von Säuren die enantiomerenreinen Ester der allgemeinen Formel (XVa,b)

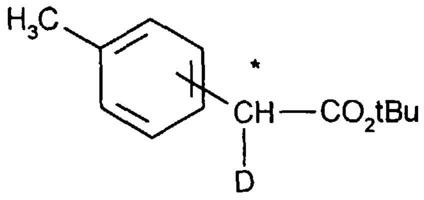

(XVa)

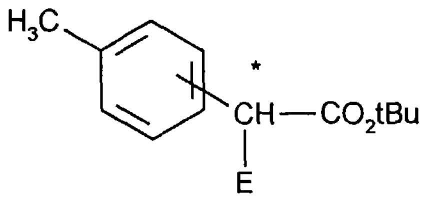

(XVb)

in welcher

D und E die oben angegebene Bedeutung haben,

herstellt,
wie oben beschrieben durch eine Halogenierung in die enantiomerenreinen Verbindungen der allgemeinen Formel (XVIa,b)

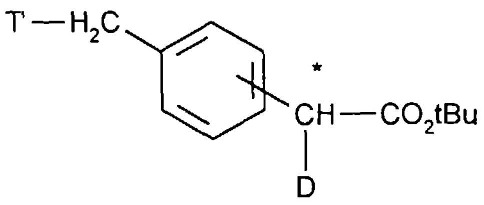

(XVIa)

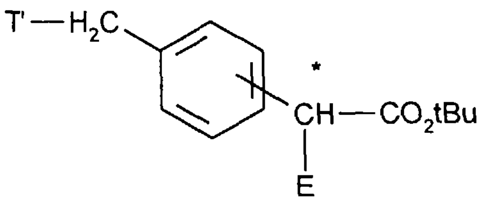

(XVIb)

in welcher

D und E die oben angegebene Bedeutung haben,
und
T' die oben angegebene Bedeutung von T hat und mit dieser gleich oder verschieden ist,

überführt,
und durch Umsetzung mit den Verbindungen der allgemeinen Formel (VI) in die enantiomerenreinen Ester der allgemeinen Formel (XVIIa,b)

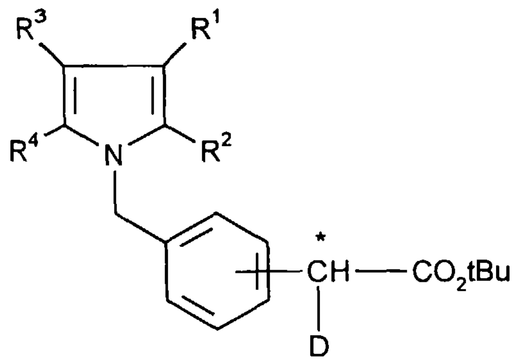

(XVIIa)

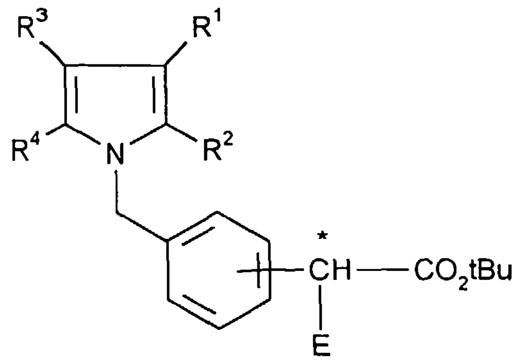

(XVIIb)

in welcher

D, E, $R^1$, $R^2$, $R^3$ und $R^4$ die oben angegebene Bedeutung haben,

überführt,
und in den letzten Schritten, wie vorne beschrieben, die entsprechenden enantiomerenreinen Säuren und deren Derivate herstellt.

Als Lösemittel für die Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Dimethylformamid, Toluol und Tetrahydrofuran.

Als Basen für die erfindungsgemäßen Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate und - hydrogencarbonate wie Natriumcarbonat, Natriumhydrogencarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl($C_1$-$C_6$)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium oder deren Hydride wie Natriumhydrid einzusetzen. Bevorzugt sind Natriumhydrogencarbonat, Kaliumcarbonat und Kalium-tert.butylat, DBU oder DABCO.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann gegebenenfalls auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Die Verseifung von tert.-Butylestern erfolgt im allgemeinen mit Säuren, wie beispielsweise Salzsäure oder Trifluoressigsäure, in Anwesenheit eines der oben angegebenen Lösemittel und/oder Wasser oder deren Gemische, vorzugsweise mit Dioxan oder Tetrahydrofuran.

Die Herstellung der Verbindungen der allgemeinen Formeln (Xa) und (Xb) erfolgt vorzugsweise in Dimethylformamid und Kalium-tert.butanolat in einem Temperaturbereich von -10°C bis +10°C.

Die Halogenierung der Verbindungen der allgemeinen Formeln (XIa) und (XIb) wird in Chlorbenzol mit 1,3-Dibrom-5,5-dimethylhydantoin in Anwesenheit von Azobisisobutyronitril in einem Temperaturbereich von 0°C bis 110°C durchgeführt.

Die Umsetzung zu den Verbindungen der allgemeinen Formeln (XIIa) und (XIIb) erfolgt unter Schutzgasatmosphäre in Dimethylformamid und Kalium-tert.butanolat in einem Temperaturbereich von 0°C bis 30°C.

Die Verseifung der Verbindungen der allgemeinen Formeln (XIIa) und (XIIb) kann wie oben beschrieben durchgeführt werden, wobei das System HBr/Ameisensäure besonders bevorzugt ist. Die Verseifung wird in einem Temperaturbereich von 20°C bis 100°C durchgeführt.

Die Umsetzung zu den Verbindungen der allgemeinen Formeln (XIIIa) und (XIIIb) erfolgt im ersten Schritt bevorzugt in Tetrahydrofuran und Triethylamin, im zweiten Schritt im System Wasser/Salzsäure. Die Reaktion wird in einem Temperaturbereich von 30°C bis 70°C durchgeführt.

Als Säure für die Herstellung der erfindungsgemäßen Verbindungen der allgemeinen Formeln (XVIa) und (XVIb) wird besonders bevorzugt konzentrierte Schwefelsäure eingesetzt. Die Herstellung wird mit Methylenchlorid durchgeführt.

Im weiteren Aufarbeitungsgang wird als Base Kaliumcarbonat eingesetzt. Die Umsetzung erfolgt in einem Temperaturbereich von 0°C bis +20°C, besonders bevorzugt bei 10°C.

Die Halogenierung der Verbindungen der allgemeinen Formeln (XVIa) und (XVIb) erfolgt mit N-Bromsuccinimid in Tetrachlorkohlenstoff in Anwesenheit von Azobisisobutyronitril.

Im allgemeinen setzt man die Base in einer Menge von 0,05 mol bis 10 mol, bevorzugt von 1 mol bis 2 mol, jeweils bezogen auf 1 mol der Verbindungen der allgemeinen Formeln (V), (IXa), (IXb), (XIIa), (XIIb), (XIIIa) und (XIIIb) ein.

Die erfindungsgemäßen Verfahren werden im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Die Verbindungen der allgemeinen Formel (III) sind neu und können wie oben beschrieben hergestellt werden.

Die Verbindungen der allgemeinen Formeln (IV), (IXa) und (IXb) sind bekannt oder können in Analogie zu bekannten Methoden hergestellt werden.

Die Verbindungen der allgemeinen Formel (VI) sind teilweise bekannt oder neu und können dann aber in Analogie zu publizierten Methoden hergestellt werden.

Die Verbindungen der allgemeinen Formel (VIII) sind als Species neu und werden aus der entsprechenden Säure hergestellt.

Die enantiomerenreinen Verbindungen der allgemeinen Formeln (Xa) und (Xb) sind mit Ausnahme von D/E = CH-isopropyl neu und können wie oben beschrieben hergestellt werden.

Die Verbindungen der allgemeinen Formeln (XIa), (XIb), (XIIa), (XIIb) sind neu und können wie oben beschrieben hergestellt werden.

Die Verbindungen der allgemeinen Formel (IV) sind an sich bekannt.

Die Verbindungen der allgemeinen Formeln (XIVa) und (XIVb) sind teilweise bekannt oder nach üblichen Methoden herstellbar.

Die enantiomerenreinen Verbindungen der allgemeinen Formeln (XVIa), (XVIb), (XVIIa) und (XVIIb) sind neu und können wie oben beschrieben hergestellt werden.

Die erfindungsgemäßen Verbindungen der allgemeinen Formel (I) haben ein nicht vorhersehbares pharmakologisches Wirkspektrum.

Sie können als Wirkstoffe in Arzneimitteln zur Reduzierung von Veränderungen an Gefäßwänden Verwendung finden und zur Behandlung von koronaren Herzerkrankungen, Herzinsuffizienz, Störungen der Hirnleistung, ischämischen Gehirnerkrankungen, Apoplexie, Durchblutungsstörungen, Mikrozirkulationsstörungen und Thrombosen.

Weiterhin spielt bei der Okklusion von Gefäßen die Proliferation glatter Muskelzellen eine ausschlaggebende Rolle. Die erfindungsgemäßen Verbindungen sind geeignet, diese Proliferation zu inhibieren und damit atherosklerotische Prozesse zu verhindern.

Die erfindungsgemäßen Verbindungen zeichnen sich durch eine Senkung der ApoB-100-assoziierten Lipoproteine (VLDL und seiner Abbauprodukte, wie z.B. LDL), des ApoB-100, der Triglyceride und des Cholesterins aus. Damit besitzen sie wertvolle, im Vergleich zum Stand der Technik überlegene pharmakologische Eigenschaften.

Überraschenderweise besteht die Wirkung der erfindungsgemäßen Verbindungen zunächst in einer Verminderung oder vollständigen Inhibierung der Bildung und/oder der Freisetzung von ApoB-100-assoziierten Lipoproteinen aus Leberzellen, was eine Senkung des VLDL-Plasmaspiegels zur Folge hat. Diese VLDL-Senkung muß mit einer Senkung der Plasmaspiegel von ApoB-100, LDL, Triglyceriden und von Cholesterin einhergehen; es werden also gleichzeitig mehrere der obengenannten Risikofaktoren gesenkt, die an Gefäßwandveränderungen beteiligt sind.

Die erfindungsgemäßen Verbindungen können daher zur Präventation und Behandlung von Atherosklerose, der Fettsucht, Pankreatitis und der Obstipation eingesetzt werden.

**1. Hemmung der Freisetzung ApoB-100-assoziierter Lipoproteine**

Der Test zum Nachweis der Hemmung der Freisetzung ApoB-100-assoziierter Liproproteine aus Leberzellen erfolgte in vitro mit kultivierten Leberzellen, bevorzugt mit Zellen der humanen Linie HepG2. Diese Zellen werden unter Standardbedingungen in Medium für die Kultur eukariontischer Zellen gezüchtet, bevorzugt in RPMI 1640 mit 10% fötalem Kälberserum. HepG2-Zellen synthetisieren und sezernieren in den Kulturüberstand ApoB-100-assoziierte Lipoproteinpartikel, die im Prinzip ähnlich aufgebaut sind wie die VLDL- bzw. LDL-Partikel, die im Plasma zu finden sind.

Diese Partikel können mit einem Immunoassay für humanes LDL nachgewiesen werden. Dieser Immunoassay erfolgt mit Antikörpern, die im Kaninchen gegen humanes LDL unter Standardbedingungen induziert worden waren. Die anti-LDL-Antikörper (Kan-anti-LDL-Ak) wurden an einem Immunosorbens mit humanem LDL affinitätschromatographisch gereinigt. Diese gereinigten Kan-anti-LDL-Ak werden an die Oberfläche von Plastik adsorbiert. Zweckmäßigerweise erfolgt diese Adsorption an die Plastikoberfläche von Mikrotitierplatten mit 96 Vertiefungen, bevorzugt an MaxiSorp-Platten. Wenn im Überstand von Hep-G2-Zellen ApoB-100-assoziierte Partikel vorhanden sind, dann können diese an die insolubilisierten Kan-anti-LDL-Ak binden, und es entsteht ein Immunkomplex, der an die Plastikoberfläche gebunden ist. Nicht gebundene Proteine werden durch Waschen entfernt. Der sich an der Plastikoberfläche befindliche Immunkomplex wird mit monoklonalen Antikörpern nachgewiesen, die nach Standardbedingungen gegen humanes LDL induziert und gereinigt worden waren. Diese Antikörper wurden mit dem Enzym Peroxidase konjugiert. Peroxidase setzt das farblose Substrat TMB in Gegenwart von $H_2O_2$ in ein gefärbtes Produkt um. Nach Ansäuerung des Reaktionsgemisches mit $H_2SO_4$ wird die spezifische Lichtabsorption bei 450 nm bestimmt, die ein Maß für die Menge von ApoB-100-assoziierten Partikeln ist, die von den HepG2-Zellen in den Kulturüberstand sezerniert worden waren.

Überraschenderweise hemmen die erfindungsgemäßen Verbindungen die Freisetzung der ApoB-100-assoziierten Partikel. Der $IC_{50}$-Wert gibt an, bei welcher Substanzkonzentration die Lichtabsorption im Vergleich zur Kontrolle (Lösemittelkontrolle ohne Substanz) um 50% inhibiert ist.

| Bsp.-Nr. | Apo B $IC_{50}$ [nM] |
|---|---|
| 2 | 8,2 |
| 20 | 12,5 |

**2. Bestimmung der VLDL-Sekretion in vivo am Hamster**

Der Effekt der Testsubstanzen auf die VLDL-Sekretion in vivo wird am Hamster untersucht. Hierzu werden Goldhamster nach Prämedikation mit Atropin (83 mg/kg s.c.) mit Ketavet (83 mg/kg s.c.) und Nembutal (50 mg/kg i.p.) narkotisiert. Wenn die Tiere reflexfrei geworden sind, wird die V. jugularis freipräpariert und kanüliert. Anschließend werden 0,25 ml/kg einer 20%igen Lösung von Triton WR-1339 in physiologischer Kochsalzlösung appliziert. Dieses Detergens hemmt die Lipoproteinlipase und führt so zu einem Anstieg des Triglyceridspiegels aufgrund eines ausbleibenden Katabolismus von sezernierten VLDL-Partikeln. Dieser Triglyceridanstieg kann als Maß für die VLDL-Sekretionsrate herangezogen werden. Den Tieren wird vor sowie ein und zwei Stunden nach Applikation des Detergens durch Punktion des retroorbitalen Venenplexus Blut entnommen. Das Blut wird zwei Stunden bei Raumtemperatur, anschließend über Nacht bei 4°C inkubiert, um die Gerinnung vollständig abzuschließen. Danach wird 5 Minuten bei 10.000 g zentrifugiert. Im so erhaltenen Serum wird die Triglyceridkonzentration mit Hilfe eines modifizierten kommerziell erhältlichen Enzymtests bestimmt (Merckotest® Triglyceride Nr. 14354). 100 µl Serum werden mit 100 µl Testreagenz in 96-Lochplatten versetzt und 10 Minuten bei Raumtemperatur inkubiert. Anschließend wird die optische Dichte bei einer

Wellenlänge von 492 nm in einem automatischen Platten-Lesegerät bestimmt (SLT-Spectra). Serumproben mit einer zu hohen Triglyceridkonzentration werden mit physiologischer Kochsalzlösung verdünnt. Die in den Proben enthaltene Triglyceridkonzentration wird mit Hilfe einer parallel gemessenen Standardkurve bestimmt. Testsubstanzen werden in diesem Modell entweder unmittelbar vor Applikation des Detergens intravenös verabreicht oder vor Einleitung der Narkose oral oder subkutan.

**3. Hemmung der intestinalen Triglyceridabsorption in vivo (Ratten)**

Die Substanzen, die auf ihre triglyceridabsorptionshemmende Wirkung in vivo untersucht werden sollen, werden männlichen Wistar-Ratten mit einem Körpergewicht zwischen 170 und 230 g oral verabreicht. Zu diesem Zweck werden die Tiere 18 Stunden vor der Substanzapplikation in Gruppen zu 6 Tieren eingeteilt und anschließend wird ihnen das Futter entzogen. Trinkwasser steht den Tieren ad libitum zur Verfügung. Die Tiere der Kontrollgruppen erhalten eine wäßrige Traganth-Suspension bzw. eine Traganth-Suspension die Olivenöl enthält. Die Traganth-Olivenöl-Suspension wird mit dem Ultra-Turrax hergestellt. Die zu untersuchenden Substanzen werden in einer entsprechenden Traganth-Olivenöl-Suspension ebenfalls mit dem Ultra-Turrax, direkt vor der Substanzapplikation suspendiert.

Jeder Ratte wird vor der Schlundsondenapplikation zur Bestimmung des basalen Serumtriglyceridgehaltes Blut durch Punktion des retroorbitalen Venenplexus entnommen. Anschließend werden die Traganth-Suspension, die Traganth-Olivenöl-Suspensionen ohne Substanz (Kontrolltiere), bzw. die Substanzen, suspendiert in einer entsprechenden Traganth-Olivenöl-Suspension, den nüchternen Tieren mit einer Schlundsonde verabreicht. Die weiteren Blutentnahmen zur Bestimmung des postprandialen Serumtriglyceridanstiegs erfolgen in der Regel 1, 2 und 3 Stunden nach der Schlundsondenapplikation.

Die Blutproben werden zentrifugiert und nach Gewinnung des Serums die Triglyceride photometrisch mit einem EPOS-Analyzer 5060 (Eppendorf Gerätebau, Netheler & Hinz GmbH, Hamburg) bestimmt. Die Bestimmung der Triglyceride erfolgt vollenzymatisch mit einem handelsüblichen UV-Test.

Der postprandiale Serumtriglyceridanstieg wird durch Subtraktion des Triglyceridvorwertes jedes Tieres von seinen korrespondierenden postprandialen Triglyceridkonzentrationen (1, 2 und 3 Stunden nach Applikation) ermittelt.

Die Differenzen (in mmol/l) zu jedem Zeitpunkt (1, 2 und 3 Stunden) werden in den Gruppen gemittelt, und die Mittelwerte des Serumtriglyceridanstiegs (ΔTG) der substanzbehandelten Tiere mit den Tieren verglichen, die nur die Traganth-Öl-Suspension erhielten.

Ebenso wird der Serumtriglyceridverlauf der Kontrolltiere, die nur Traganth erhielten, berechnet. Der Substanzeffekt zu jedem Zeitpunkt (1, 2 oder 3 Stunden) wird wie folgt ermittelt und in Δ% von der ölbelasteten Kontrolle angegeben.

$$\Delta\%\ \text{Triglyceridanstieg} = \frac{\Delta TG_{\text{Substanz}} - \Delta TG_{\text{Traganthkontrolle}}}{\Delta TG_{\text{Ölbestlastung}} - \Delta TG_{\text{Traganthkontrolle}}} \times 100$$

Effekt von 1, 3 oder 10 mg Prüfsubstanz / kg KG p.o. auf den Triglyceridanstieg (Δ%) 2 h nach einer Triglyceridbelastung im Serum nüchterner Ratten. Der Serumtriglyceridanstieg fettbelasteter Kontrolltiere bezogen auf den Serumtriglyceridspiegel von Traganth-Kontrolltieren entspricht 100%. n = 6 Tiere pro Gruppe.

| | **Serumtriglyceridanstieg in % (2 h pp)** |
|---|---|
| **Triglyceridbelastung** | 100 |
| **Traganthkontrolle** | 0 |

Die statistische Auswertung erfolgt mit Student's t-Test nach vorheriger Überprüfung der Varianzen auf Homogenität.

Substanzen, die zu einem Zeitpunkt den postprandialen Serumtriglyceridanstieg, verglichen mit dem der unbehandelten Kontrollgruppe, statistisch signifikant ($p < 0{,}05$) um mindestens 30 % vermindern, werden als pharmakologisch wirksam angesehen.

**4. Hemmung der VLDL-Sekretion in vivo (Ratte)**

Die Wirkung der Testsubstanzen auf die VLDL-Sekretion wird ebenfalls an der Ratte untersucht. Dazu wird Ratten

500 mg/kg Körpergewicht Triton WR-1339 (2,5 ml/kg), gelöst in physiologischer Kochsalzlösung, intravenös in die Schwanzvene appliziert. Triton WR-1339 inhibiert die Lipoproteinlipase und führt somit durch Hemmung des VLDL-Katabolismus zu einem Anstieg des Triglycerid- und Cholesterinspiegels. Diese Anstiege können als Maß für die VLDL-Sekretionsrate herangezogen werden.

Den Tieren wird vor sowie zwei Stunden nach Applikation des Detergens durch Punktion des retroorbitalen Venenplexus Blut entnommen. Das Blut wird zur Gerinnung 1 h bei Raumtemperatur inkubiert und das Serum durch Zentrifugation mit 10 000 g für 20 s gewonnen. Anschließend werden die Triglyceride mittels eines handelsüblichen gekoppelten Enzymtests (Sigma Diagnostics®, Nr. 339) bei einer Wellenlänge von 540 nm photometrisch bestimmt. Die Messung erfolgt mit Hilfe eines ebenfalls gekoppelten Enzymtests (Boehringer Mannheim®, Nr. 1442350) bei einer Wellenlänge von 546 nm. Proben mit Triglycerid- bzw. Cholesterinkonzentrationen, die den Meßbereich der Methoden überschreiten, werden mit physiologischer Kochsalzlösung verdünnt. Die Ermittlung der jeweiligen Serumkonzentrationen erfolgt anhand parallel gemessener Standardreihen. Testsubstanzen werden unmittelbar nach der Tritoninjektion oral, intravenös oder subcutan appliziert.

Die Erfindung betrifft außerdem die Kombination von neuen Arylessigsäureamiden der allgemeinen Formel (I) mit einem Glucosidase- und/oder Amylasehemmer zur Behandlung von familiärer Hyperlipidaeamien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, Vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen Von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der Vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Verwendete Abkürzungen:

| | |
|---|---|
| Ac = | Acetyl |
| Bn = | Benzyl |
| Bz = | Benzoyl |
| iBu = | iso Butyl |
| nBu = | normal Butyl |
| sBu = | sekundär Butyl |
| tBu = | tertiär Butyl |
| DDQ = | 2,3-Dichlor-5,6-dicyano-1,4-benzochinon |
| cDec = | cyclo-Decyl |
| DMF = | N,N-Dimethylformamid |
| DMSO = | Dimethylsulfoxid |
| cDodec = | cyclo-Dodecyl |
| Et = | Ethyl |
| cHept = | cyclo-Heptyl |

| | |
|---|---|
| cHex = | cyclo-Hexyl |
| HOBT = | 1-Hydroxy-1H-benzotriazol |
| Me = | Methyl |
| Mes = | Mesyl |
| cNon = | cyclo-Nonyl |
| cOct = | cyclo-Octyl |
| cPent = | cyclo-Pentyl |
| nPent = | normal Pentyl |
| Ph = | Phenyl |
| cPr = | cyclo-Propyl |
| nPr = | normal Propyl |
| iPr = | iso Propyl |
| THF = | Tetrahydrofuran |
| TMS = | Tetramethylsilan |
| pTol = | para Tolyl |
| pTos = | para Tosyl |
| cUndec = | cyclo-Undecyl |

| Solvens | Bezeichnung |
|---|---|
| Dichlormethan : Methanol = 20:1 | A |
| Dichlormethan : Methanol = 50:1 | B |
| Dichlormethan : Ethanol = 20:1 | C |
| Dichlormethan : Ethanol = 50:1 | D |
| Petrolether : Essigsäureethylester = 1:1 | E |
| Dichlormethan : Methanol : Essigsäure = 90:10:2 | F |
| Petrolether : Essigsäureethylester = 2:1 | G |
| Petrolether : Essigsäureethylester = 10:1 | H |
| Toluol | I |
| Toluol : Essigsäureethylester = 1:1 | K |
| Petrolether : Essigsäureethylester = 5:1 | L |
| Dichlormethan | M |
| Petrolether : Essigsäureethylester = 20:1 | N |
| Dichlormethan : Methanol = 10:1 | O |
| Cyclohexan : Essigsäureethylester = 1:1 | P |
| Toluol : Essigsäureethylester = 9:1 | Q |
| Toluol : Essigsäureethylester = 8:1 | R |
| Petrolether : Essigsäureethylester = 1:2 | S |
| Dichlormethan : Ethanol = 5:1 | T |
| Dichlormethan : Ethanol = 10:1 | U |
| Petrolether : Essigsäureethylester = 9:1 | V |
| Dichlormethan : Methanol = 19:1 | W |
| Petrolether : Essigsäureethylester = 4:1 | X |
| Dichlormethan : Methanol = 100:1 | Y |
| Dichlormethan : Methanol = 100:3 | Z |
| Petrolether : Essigsäureethylester = 6:1 | XA |

**Zubereitungsvorschrift für das DC-Laufmittel BABA:**

87,9 ml einer wäßrigen 0,06667 molaren Kaliumdihydrogenphosphatlösung und 12,1 ml einer wäßrigen 0,06667 molaren Dinatriumhydrogenphosphatlösung werden gemischt. 60 ml der so zubereiteten Lösung werden mit 200 ml n-Butylacetat, 36 ml n-Butanol und 100 ml Eisessig geschüttelt und die wäßrige Phase abgetrennt. Die organische Phase ist das Laufmittel BABA.

**Ausgangsverbindungen**

**Beispiel I**

6-Chlor-2,4-lutidin

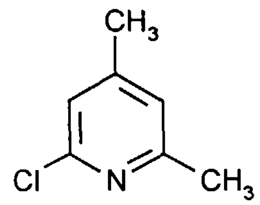

Zur Darstellung der Titelverbindung [US 36 32 807] werden 600 g (4,91 mol) 6-Amino-2,4-lutidin in 2 l Methanol gelöst und die Lösung bei ca. 0°C mit Chlorwasserstoffgas gesättigt. Bei einer Innentemperatur unter 10°C werden 1,307 l (9,82 mol) Isopentylnitrit zugetropft (ca. 2,5 h) und das Gemisch unter Erwärmung auf Raumtemperatur (ca. 25°C) 15 h so belassen. Die Lösung wird im Vakuum weitgehend vom Lösemittel befreit, mit 3 l Dichlormethan und 1,5 l Wasser versetzt und unter Kühlung (< 20°C) mit konzentrierter wäßriger Ammoniaklösung auf einen pH = 9,5 gestellt. Die abgetrennte organische Phase wird mit Natriumsulfat getrocknet, zuerst am Rotationsverdampfer im Vakuum eingeengt und dann über eine Vigreux-Kolonne destilliert:

Fraktion 1) Kp. = 47-49°C (12 Torr), 603 g
Fraktion 2) Kp. = 82-85°C (12 Torr), 612 g (ca. 88% roh)
$R_f$ = 0,39 (Petrolether : Essigester = 10:1)
$^1$H-NMR ($CDCl_3$, 200 MHz, TMS): $\delta$ = 2,28 (s, 3H), 2,47 (s, 3H), 6,88 (s, 1H), 6,96 (s, 1H) ppm.

Das rohe Produkt, das kleine Mengen 6-Methoxy-2,4-lutidin enthalten kann, wird ohne weitere Reinigung weiter umgesetzt.

**Beispiel II**

6-Hydrazino-2,4-lutidin (4,6-Dimethyl-2-hydrazino-pyridin)

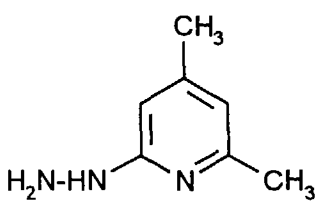

580 g (4,10 mol) der Verbindung aus Beispiel II werden in 800 ml Diethylenglycol gelöst und mit 1050 ml Hydrazin-Hydrat 48 h bei einer Badtemperatur von ca. 140°C gerührt. Der abgekühlte Ansatz wird auf 4,5 l Ether und 4,5 l Wasser gegossen und die organische Phase wird zweimal mit je 2,3 l Dichlormethan extrahiert. Die vereinigten organischen Phasen werden mit Natriumsulfat getrocknet und im Vakuum eingedampft. Es fallen 784 g lösemittelhaltiges Rohprodukt an, das ohne Aufarbeitung weiter umgesetzt wird.

$R_f \simeq$ 0,37 (Dichlormethan : Methanol = 10:1)
$^1$H-NMR ($d_6$-DMSO, 250 MHz, TMS): $\delta$ = 2,13 (s, 3H), 2,22 (s, 3H), 4,02 (s, 2H), 6,26 (s, 1H), 6,35 (s, 1H), 7,11 (s, 1H) ppm.

**Beispiel III**

2,4-Dimethyl-5,6,7,8-tetrahydro-α-carbolin

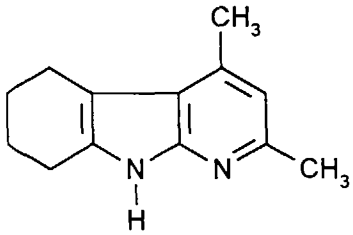

78 g (max. 0,49 mol) rohe Verbindung aus Beispiel II werden bei Raumtemperatur (ca. 25°C) mit 59 ml (0,56 mol) Cyclohexanon umgesetzt, wobei die Innentemperatur ansteigt. Nach 2 h ist das Edukt verschwunden (DC-Kontrolle; Dichlormethan : Methanol = 10:1). Die Mischung wird in 40 ml Diethylenglycol aufgenommen und unter Rückfluß umgesetzt, dabei werden tiefer als das Lösemittel siedende Bestandteile (z.B. Reaktionswasser und überschüssiges Cyclohexanon) destillativ entfernt (Wasserabscheider). Nach 3 h ist das intermediäre Hydrazon verschwunden (DC-Kontrolle; Petrolether : Essigester = 1:1); das Reaktionsgemisch wird auf Raumtemperatur abgekühlt und mit Aceton verrührt. Der anfallende Niederschlag wird abgesaugt, mit Aceton nachgewaschen und im Vakuum getrocknet (34,4 g). Die vom Lösemittel weitgehend befreiten Mutterlaugen werden wiederum mit Aceton behandelt, wobei weitere 9,3 g Produkt anfallen (Gesamtausbeute über 3 Stufen: 43,7 g / 0,22 mol / 47%).

Schmp.: 248°C (unkorrigiert)
$R_f$ = 0,41 (Dichlormethan : Ethanol = 20:1)
$^1$H-NMR ($d_6$-DMSO, 200 MHz, TMS): δ = 1,78 (m, 4H), 2,40 (s, 3H), 2,48 (s, 3H), 2,64 (m, 2H), 2,82 (m, 2H), 6,57 (s, 1H), 10,84 (s, 1H) ppm.

Die Verbindungen der Tabelle I werden analog der Vorschrift von Beispiel III hergestellt:

**Tabelle I:**

| Bsp.-Nr. | | $R_f$ (Solvens) | Ausgangsmaterial (Hydrazin *) |
|---|---|---|---|
| IV | 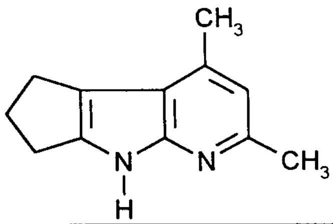 | 0,59 (A) | Bsp.-Nr. II |
| V | 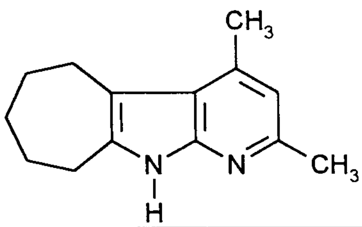 | 0,36 (E) | Bsp.-Nr. II |
| VI | 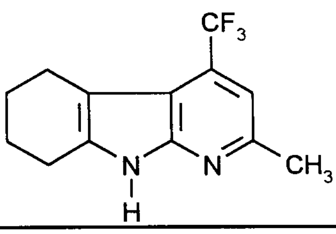 | 0,45 (G) | |
| VII | 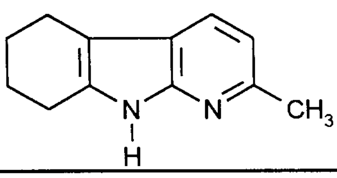 | 0,46 (E) | |

| Bsp.-Nr. | | $R_f$ (Solvens) | Ausgangsmaterial (Hydrazin *) |
|---|---|---|---|
| VIII | 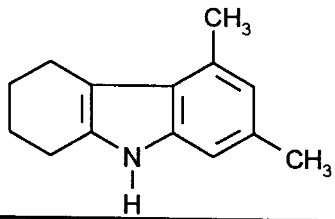 | 0,06 (L) | |
| IX | 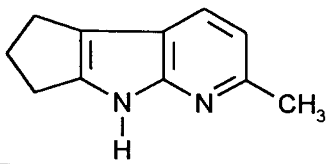 | 0,41 (E) | |
| X | 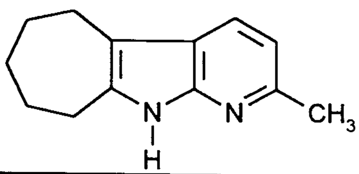 | 0,40 (E) | |
| XI | 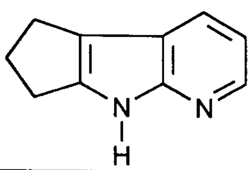 | 0,59 (O) | |
| XII | 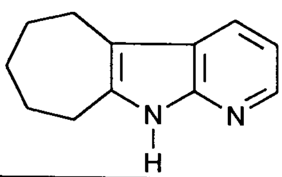 | 0,34 (E) | |
| XIII | 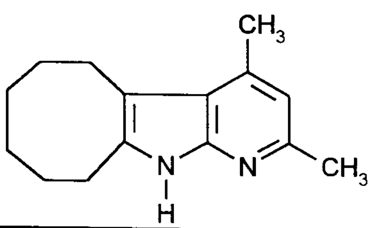 | 0,42 (E) | |
| XIV | 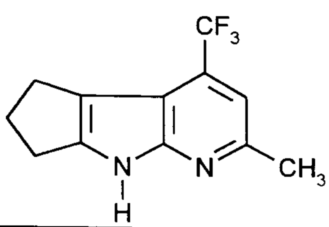 | 0,59 (G) | |

| Bsp.-Nr. | | $R_f$ (Solvens) | Ausgangsmaterial (Hydrazin *) |
|---|---|---|---|
| XV | 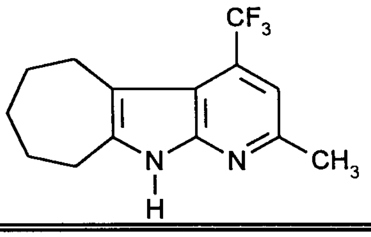 | 0,85 (G) | |

**Beispiel XVI**

2,4-Dimethyl-α-carbolin

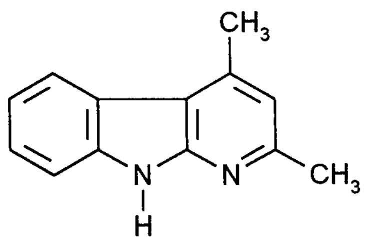

100 g (499 mmol) der Verbindung aus Beispiel III werden in 700 ml Diethylenglykol mit 164 ml (1 mol) Fumarsäurediethylester an 52 g Palladium (5% auf Kohle) unter Rückfluß umgesetzt. Bei der hohen Innentemperatur destilliert eine kleine Menge Ethanol ab (ggfs. Wasserabscheider verwenden). Nach ca. 8 h ist das Edukt verschwunden (DC-Kontrolle; Petrolether : Essigester = 1:1, Detektion in der Jodkammer). Das abgekühlte Gemisch wird mit 3 l Aceton versetzt, aufgekocht, heiß über einen Klärfilter (Fa. Seitz) abgesaugt und mit 1 l heißem Aceton nachgewaschen. Beim Abkühlen fällt ein Niederschlag an, der nach Absaugen, Spülen mit kaltem Aceton und Trocknen im Vakuum 58,3 g Produkt liefert. Die Mutterlauge wird im Vakuum weitgehend vom Aceton befreit, wobei der ausfallende Niederschlag wie oben aufgearbeitet wird (9,4 g). Das Filtrat wird wiederum vom Aceton befreit; nach Zugabe von n-Pentan fällt ein weiteres Mal Produkt aus (3,1 g / Aufarbeitung s.o.); Gesamtausbeute: 72%.

Schmp.: 220-221°C (unkorrigiert)
$R_f$ = 0,47 (Petrolether : Essigester = 1:1)
$^1$H-NMR ($d_6$-DMSO, 200 MHz, TMS): $\delta$ = 2,54 (s, 3H), 2,75 (s, 3H), 6,89 (s, 1H), 7,20 (m, 1H), 7,40 (m, 1H), 7,48 (dd, 1H), 8,05 (dd, 1H), 11,61 (s, 1H) ppm.

**Beispiel XVII**

2(R,S)-2-Cyclopentyl-2-[4-(2,4-dimethyl-α-carbolin-9-yl)methyl]phenyl-essigsäure-tert.butylester

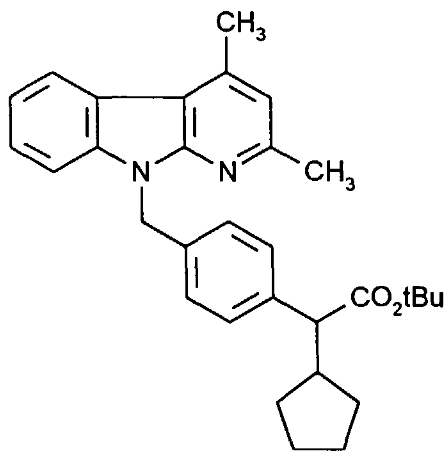

73,6 g (375 mmol) der Verbindung aus Beispiel XVI werden bei 25°C in 700 ml wasserfreiem N,N-Dimethylformamid mit 42,13 g (375 mmol) Kalium-tert.butanolat 30 min. umgesetzt und dann mit 161,7 g (375 mmol) der Verbindung aus Beispiel XXVIII, gelöst in 680 ml wasserfreiem N,N-Dimethylformamid, versetzt. Nach 1 h ist die Umsetzung beendet (DC-Kontrolle; Petrolether : Essigester = 10:1). Zur Aufarbeitung werden 2 l Pufferlösung (pH = 4 / Fa. Merck) und 2 l Wasser zugegeben, der anfallende Niederschlag abgesaugt, mit Wasser gewaschen und wieder scharf abgesaugt. Der mäßig feuchte Feststoff wird nun nacheinander mit Petrolether und Methanol verrührt und abgesaugt. Die Vakuumtrocknung über Phosphorpentoxid liefert 139,8 g (298 mmol / 79%) Produkt.

Schmp.: 160-161°C (unkorrigiert)
$R_f$ = 0,39 (Petrolether : Essigester = 10:1)
$^1$H-NMR ($CDCl_3$, 250 MHz, TMS): δ = 0,91 (m, 1H), 1,18-1,68 (m, 6H), 1,87 (m, 1H), 1,47 (s, 9H), 2,42 (m, 1H), 2,66 (s, 3H), 2,83 (s, 3H), 3,09 (d, 1H), 5,67 (s, 2H), 6,88 (s, 1H), 7,13-7,41 (m, 7H), 8,09 (d, 1H) ppm

Die Verbindungen der Tabellen II und III werden analog der Vorschrift des Beispiels XVIII hergestellt:

**Tabelle II:**

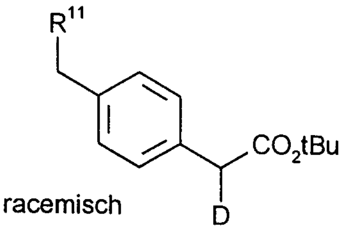

racemisch

| Bsp.-Nr. | $R^{11}$ | D | $R_f$ (Solvens) |
|---|---|---|---|
| XVIII | 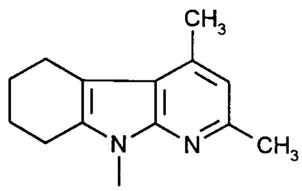 | cPent | 0,28 (H) |
| XIX | 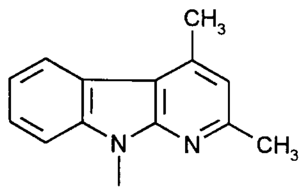 | cHept | 0,47 (H) |
| XX | 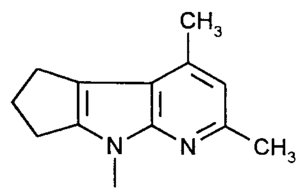 | cHept | 0,54 (L) |
| XXI | 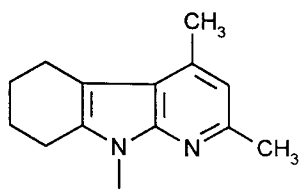 | cHept | 0,27 (H) |
| XXII | 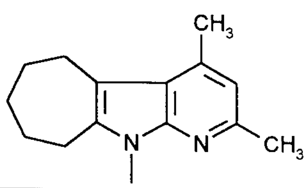 | cPent | 0,59 (D) |
| XXIII | 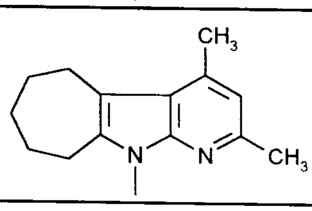 | cHept | 0,29 (H) |

| Bsp.-Nr. | $R^{11}$ | D | $R_f$ (Solvens) |
|---|---|---|---|
| XXIV | 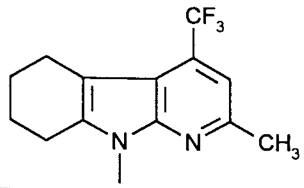 | cPent | 0,70 (M) |
| XXV | 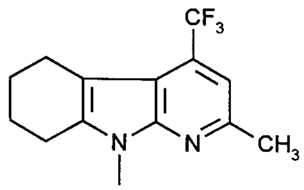 | cHept | 0,36 (H) |
| XXVI | 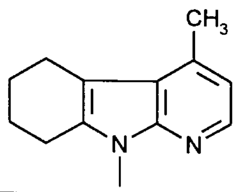 | cHept | 0,48 (L) |
| XXVII | 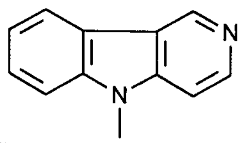 | cPent | 0,49 (C) |
| XXVIII | 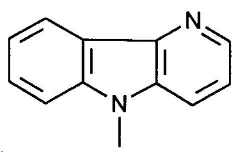 | cPent | 0,51 (C) |
| XXIX | 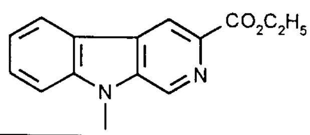 | cPent | 0,54 (C) |
| XXX | 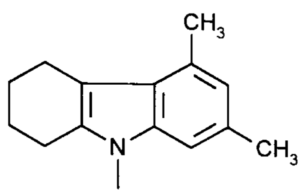 | cPent | 0,37 (N) |
| XXXI | 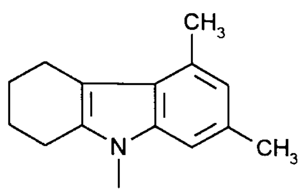 | cHept | 0,56 (H) |

| Bsp.-Nr. | $R^{11}$ | D | $R_f$ (Solvens) |
|---|---|---|---|
| XXXII | 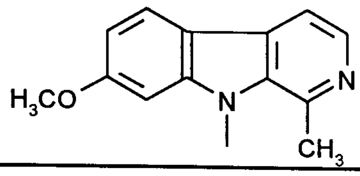 | cPent | 0,57 (C |
| XXXIII | 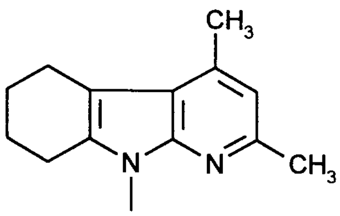 | cHex | 0,35 (H) |
| XXXIV | 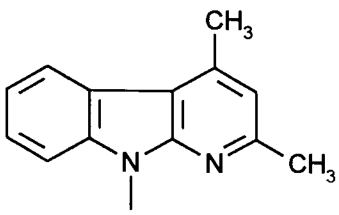 | cHex | 0,57 (B) |
| XXXV | 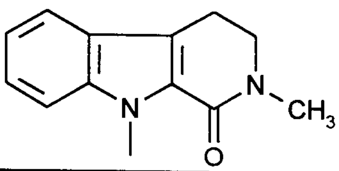 | cPent | Fp.= 189-190°C |
| XXXVI | 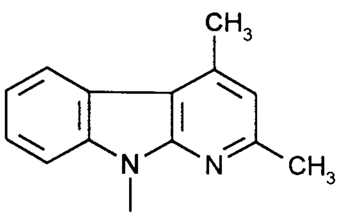 | iBu | 0,49 (M)<br>Fp.: 142°C<br>MS (CI/$NH_3$):<br>457 (100%) |

**Tabelle III:**

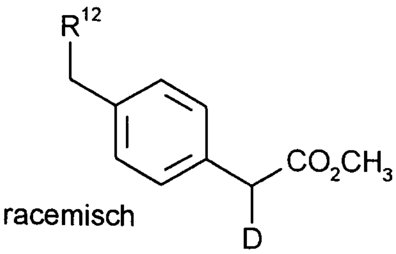

racemisch

| Bsp.-Nr. | $R^{12}$ | D | $R_f$ (Solvens) MS/Fp. |
|---|---|---|---|
| XXXVII | 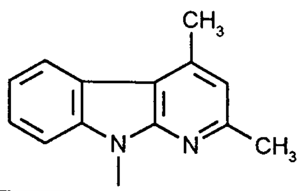 | iPr | 0,39 (M)<br>Fp:= 159°C<br>MS(CI/$NH_3$):<br>401 (100%) |
| XXXVIII | 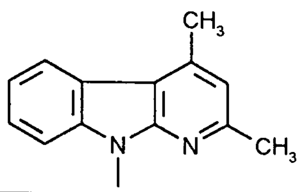 | cPent | 0,76 (B) |
| XXXIX | 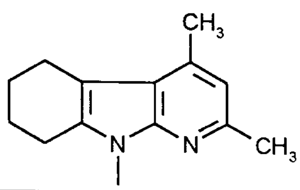 | cHept | 0,26 (H) |
| XL | 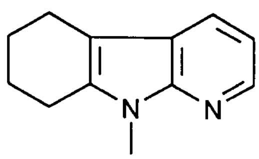 | cHept | 0,64 (K) |
| XLI | 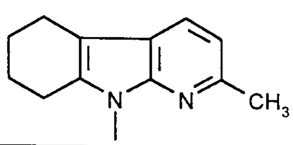 | cHept | 0,29 (H) |
| XLII | 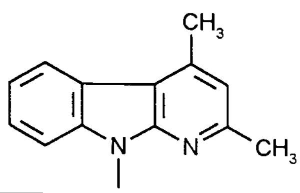 | cHept | 0,30 (H) |

**Beispiel XLIII**

2-(R,S)-2-Cyclopentyl-2-[4-(2,4-dimethyl-α-carbolin-9-yl)methyl]phenylessigsäure Hydrochlorid

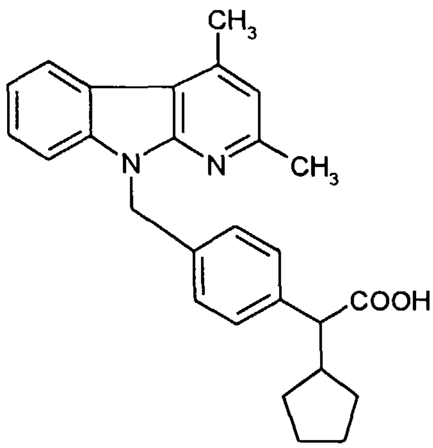

x HCl

139,8 g (298 mmol) der Verbindung aus Beispiel XVII werden in 1 l 1,4-Dioxan gelöst und 3 h mit 240 ml konzentrierter Salzsäure (37%ig) bei 70°C gerührt. Nach beendeter Umsetzung (DC-Kontrolle; Petrolether : Essigester = 10:1) wird der Ansatz auf ca. 15°C gekühlt und dann portionsweise auf 5 l Wasser gegossen. Der pH-Wert wird mit 2 M wäßriger Natronlauge auf 2,8 gestellt, der anfallende Niederschlag über einen Papierfilter abgesaugt und mit Wasser so oft nachgewaschen, bis das Waschwasser einen pH > 4 besitzt. Der scharf abgesaugte Feststoff wird mit 1 l Petrolether (Siedebereich 60-80°C) verrührt, wiederum abgesaugt und im Vakuum über Phosphorpentoxid getrocknet.

Ausbeute: 130,3 g (290 mmol / 97%)
Schmp.: 260-262°C (unkorrigiert)
$R_f$ = 0,51 (Dichlormethan : Ethanol = 20:1)
$^1$H-NMR ($d_6$-DMSO, 200 MHz, TMS): $\delta$ = 0,88 (m, 1H), 1,09-1,67 (m, 6H), 1,79 (m, 1H), 2,38 (m, 1H), 2,68 (s, 3H), 2,84 (s, 3H), 3,16 (d, 1H), 4,7-5,9 (1H), 5,80 (s, 2H), 7,12-7,26 (m, 5H), 7,32 (m, 1H), 7,49 (m, 1H), 7,59 (d, 1H), 8,17 (d, 1H) ppm.

Die Verbindungen der Tabelle IV werden analog der Vorschrift des Beispiels XLIII hergestellt:

**Tabelle IV:**

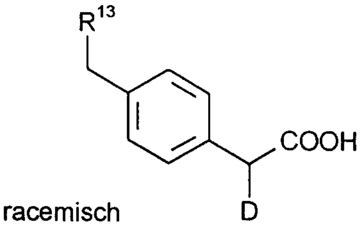

racemisch

| Bsp.-Nr. | $R^{13}$ | D | $R_f$ (Solvens) |
|---|---|---|---|
| XLIV | 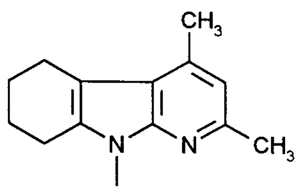 | cPent | 0,37 (A) |
| XLV | 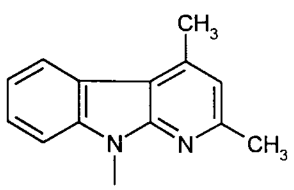 | cHept | 0,23 (G) |
| XLVI | 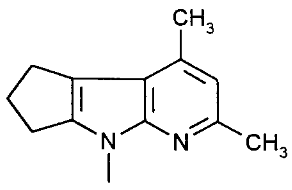 | cHept | 0,30 (E) |
| XLVII | 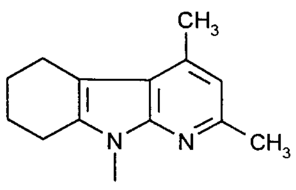 | cHept | 0,27 (D) |

| Bsp.-Nr. | $R^{13}$ | D | $R_f$ (Solvens) |
|---|---|---|---|
| XLVIII | 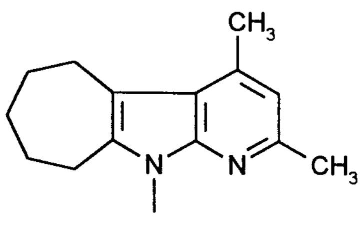 | cPent | 0,37 (C) |
| XLIX | 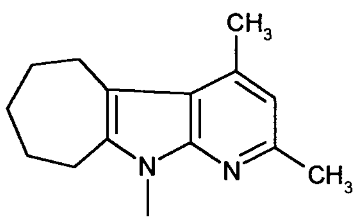 | cHept | 0,15 (C) |
| L | 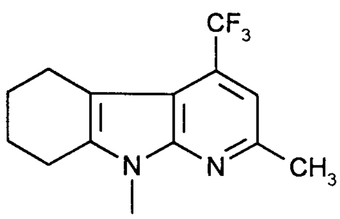 | cPent | 0,43 (A) |
| LI | 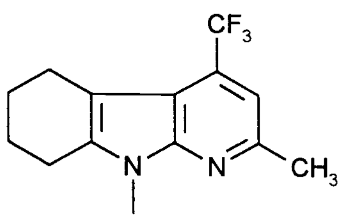 | cHept | 0,27 (C) |

| **Bsp.-Nr.** | **$R^{13}$** | **D** | **$R_f$ (Solvens)** |
|---|---|---|---|
| LII | 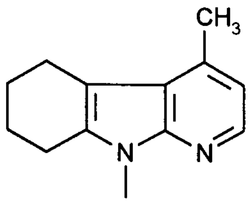 | cHept | 0,17 (E) |
| LIII | 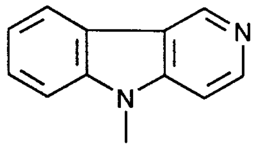 | cPent | 0,07 (C) |
| LIV | 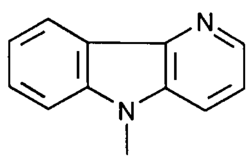 | cPent | 0,26 (C) |
| LV | 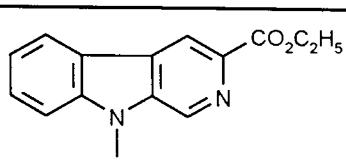 | cPent | 0,39 (C) |

| **Bsp.-Nr.** | **$R^{13}$** | **D** | **$R_f$ (Solvens)** |
|---|---|---|---|
| LVI | 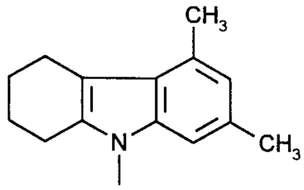 | cPent | 0,46 (C) |
| LVII | 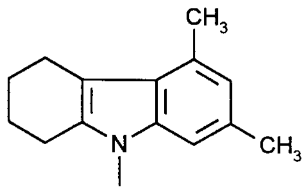 | cHept | 0,68 (E) |
| LVIII | 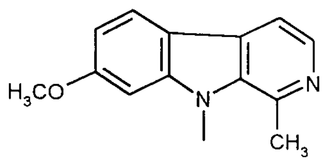 | cPent | 0,44 (C) |
| LIX | 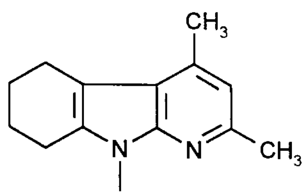 | cHex | 0,44 (C) |
| LX | 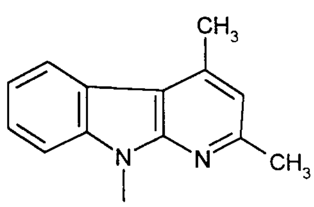 | cHex | 0,55 (C) |

| Bsp.-Nr. | $R^{13}$ | D | $R_f$ (Solvens) |
|---|---|---|---|
| LXI | 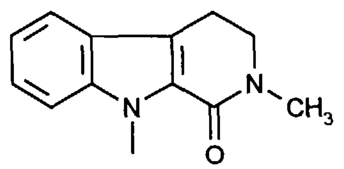 | cPent | Fp. 204-205°C |
| LXII | 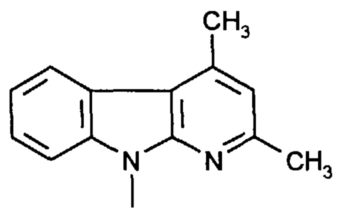 | iBu | 0,36 (A)<br>Fp.: 156°C<br>MS(FAB):<br>401(100%)<br>154 (90%) |

Beispiel LXIII

4-Tolyl-essigsäure-methylester

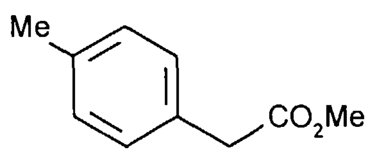

300 g (1,998 mol) 4-Tolyl-essigsäure werden in 2,5 l Methanol gelöst, mit 100 ml konzentrierter Schwefelsäure verrührt und 2,5 Stunden unter Rückfluß gekocht. Darauf rührt man nach und nach insgesamt 430 g (5,1 mol) Natriumhydrogencarbonat ein (Kohlendioxidentwicklung !), dampft das Methanol im Vakuum weitgehend ab, verteilt zwischen Wasser und Dichlormethan und extrahiert die wäßrige Phase mit Dichlormethan nach. Die vereinigten organischen Phasen werden mit Natriumsulfat getrocknet und im Vakuum vom Lösemittel befreit. Der Rückstand wird im Hochvakuum destiliert.

Ausbeute: 336 g
Siedetemperatur = 65°C (0,5 mbar)
$R_f$ = 0,81 (Toluol : Essigsäureethylester = 2:1)

**Beispiel LXIV**

4-Tolyl-essigsäureethylester

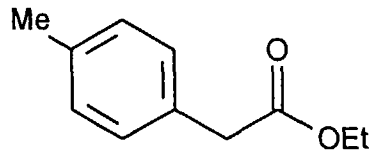

Ausgehend von 4-Tolyl-essigsäure wird der 4-Tolyl-essigsäureethylester analog der Vorschrift aus Beispiel LXIII hergestellt.

$R_f$ = 0,43 (N)

**Beispiel LXV**

4-Methylphenylessigsäure-tert.butylester

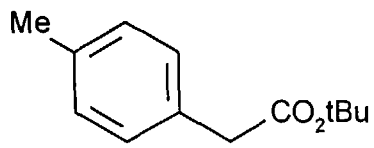

450 g (3 mol) 4-Methylphenylessigsäure, 1,13 l (12 mol) tert.Butanol und 90 g (0,74 mol) Dimethylaminopyridin werden in 2 l Dichlormethan gelöst. Nach Zugabe von 680 g (3,3 mol) Dicyclohexylcarbodiimid, gelöst in 400 ml Dichlormethan, wird 20 h bei 25°C gerührt, der ausgefallene Harnstoff abgesaugt, mit 200 ml Dichlormethan gewaschen und die organische Phase je zweimal mit 500 ml 2M Salzsäure und Wasser gewaschen. Die organische Phase wird mit Natriumsulfat getrocknet, eingeengt und destilliert.

Ausbeute: 408 g (66%)
Siedepunkt: 73-78°C (0,2 Torr)

**Beispiel LXVI**

2-Cyclopentyl-2-(4-methylphenyl)essigsäure-tert.butylester

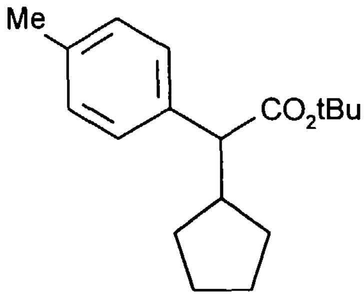

33,5 g (0,3 mol) Kalium-tert.butylat werden in 100 ml DMF unter Feuchtigkeitsausschluß bei 0°C vorgelegt, und 51,6 g (0,25 mol) der Verbindung aus Beispiel LXV in 250 ml DMF zugetropft. Es wird 30 min bei 0°C gerührt, 32,2 ml (0,3 mol) Cyclopentylbromid in 150 ml DMF bei 5-15°C zugetropft und 20 h bei 25°C gerührt. Nach Einengen wird der Rückstand zwischen Wasser / Diethylether verteilt, die Etherphase über Natriumsulfat getrocknet und eingeengt. Das Produkt kristallisiert aus.

Ausbeute: 67 g (97,5%)
Festpunkt: 51-53°C

Die Verbindungen der Tabelle III werden analog der Vorschrift des Beispiels LXVI hergestellt:

**Tabelle III:**

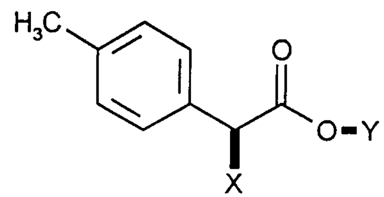

| Bsp.-Nr. | —X | —Y | a) Fp. (°C) b) $R_f$ (Solvens) | Spektren | Ausgangsmaterial aus Bsp.-Nr. |
|---|---|---|---|---|---|
| LXVII | —$CH_3$ = Me | —$C(CH_3)_3$ = tBu | b) 0,71 (I) | | LXV |
| LXVIII | —$C_2H_5$ = Et | tBu | b) 0,67 (I) | | LXV |
| LXIX | —$CH_2CH_2CH_3$ = nPr | tBu | b) 0,69 (I) | | LXV |
| LXX | —$CH(CH_3)_2$ = iPr | Me | b) 0,86 (Toluol : Essigester = 9:1) | | LXIII |
| LXXI | —$CH(CH_3)_2$ = iPr | tBu | b) 0,76 (Q) | | LXV |
| LXXII | —$CH_2CH_2CH_2CH_3$ = nBu | tBu | b) 0,74 (I) | | LXV |
| LXXIII | —$CH_2CH(CH_3)_2$ = iBu | tBu | b) 0,70 (I) | | LXV |
| LXXIV | —$CH_2CH_2CH_2CH_2CH_3$ = nPent | tBu | b) 0,75 (V) | | LXV |
| LXXV | —$CH_2CH_2$-$CH(CH_3)_2$ = iPent | tBu | b) 0,54 (Petrolether : Essigester = 10:1) | | LXV |

| Bsp.-Nr. | —X | —Y | a) Fp. (°C)<br>b) $R_f$ (Solvens) | Spektren | Ausgangsmaterial aus Bsp.-Nr. |
|---|---|---|---|---|---|
| LXXVI | —$CH(CH_2CH_3)_2$ | tBu | | MS: 276 ($M^+$, 4%) | LXV |
| LXXVII | —$CH_2CH_2CH_2CH_2CH_2CH_3$ = nHex | tBu | b) 0,75 (I) | | LXV |
| LXXVIII | —$CH_2CH(CH_2CH_3)_2$ | tBu | | MS: 290 ($M^+$, 1%) | LXV |
| LXXIX | 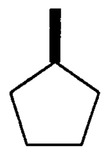 = cPent | Me | b) 0,59 (Petrolether : Essigester = 10:1) | | LXIII |
| LXXX | 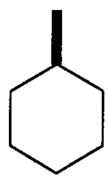 = cHex | Me | b) 0,62 (Petrolether : Essigester = 10:1) | | LXIII |
| LXXXI | cHex | tBu | b) 0,72 (I) | | LXV |
| LXXXII | 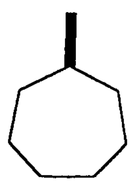 = cHept | Me | b) 0,57 (I) | | LXIII |
| LXXXIII | cHept | tBu | b) 0,67 (I) | | LXV |

| Bsp.-Nr. | X | Y | a) Fp. (°C) b) $R_f$ (Solvens) | Spektren | Ausgangsmaterial aus Bsp.-Nr. |
|---|---|---|---|---|---|
| LXXXIV | 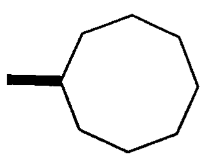 = cOct | tBu | b) 0,77 (I) | | LXV |
| LXXXV | 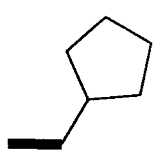 | tBu | (H) | | LXV |
| LXXXVI | 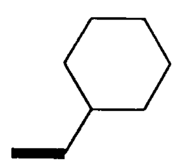 | tBu | b) 0,82 (Q) | | LXV |

Die Verbindungen der Tabelle IV werden analog der Vorschrift von Beispiel LXVI hergestellt; es werden lediglich 2,5 Äquivalente der Base und 2,5 Äquivalente des Halogenalkans (im Falle der cyclischen Alkylreste 1,2 Äquivalente des $\alpha,\omega$-Dihalogenalkans) eingesetzt.

**Tabelle IV**

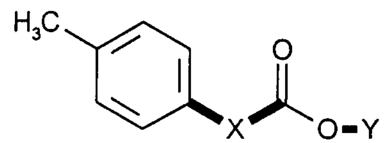

| Bsp.-Nr. | 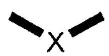 | 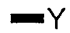 | a) Fp. (°C)<br>b) $R_f$ (Solvens) | Spektren | Ausgangsmaterial aus Bsp.-Nr. |
|---|---|---|---|---|---|
| LXXXVII | 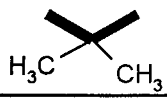 | tBu | b) 0,68 (F) | | LXV |
| LXXXVIII | 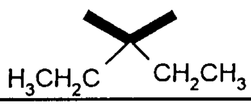 | tBu | b) 0,32 (Petrolether) | | LXV |
| LXXXIX | 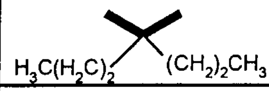 | tBu | b) 0,84 (B) | | LXV |
| XC | 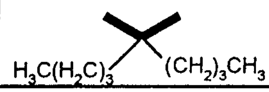 | tBu | b) 0,82 (C) | | LXV |

| Bsp.-Nr. | 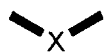 | 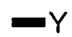 | a) Fp. (°C)<br>b) $R_f$ (Solvens) | Spektren | Ausgangsmaterial aus Bsp.-Nr. |
|---|---|---|---|---|---|
| XCI | 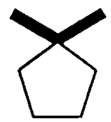 | tBu | b) 0,23 (Petrolether) | | LXV |
| XCII | 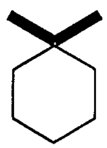 | tBu | b) 0,21 (Petrolether) | | LXV |
| XCIII | 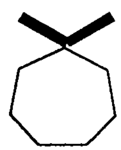 | tBu | b) 0,26 (Petrolether) | | LXV |

**Beispiel XCIV**

2-(4-Brommethyl-phenyl)-2-cyclopentyl-essigsäure-tert.butylester

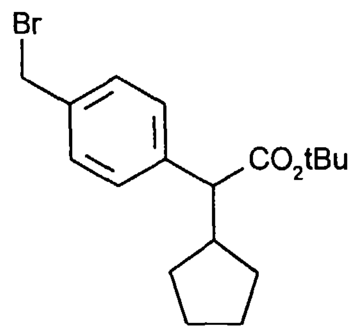

27,4 g (0,1 mol) der Verbindung aus Beispiel LXV werden in 200 ml Tetrachlorkohlenstoff gelöst und zum Sieden erhitzt. Nach Zugabe von 0,82 g Azobisisobutyronitril werden 18,7 g (0,105 mol) N-Bromsuccinimid portionsweise zugegeben und anschließend 1 h refluxiert, auf 0°C abgekühlt und vom Succinimid abfiltriert. Nach Einengen des Filtrates fällt das Produkt aus. Es wird mit Petrolether (40/60) gewaschen und getrocknet.

Ausbeute: 20 g (57%)
Festpunkt: 73-76°C

Die Verbindungen der Tabelle V werden analog der Vorschrift von Beispiel-Nr. XCIV hergestellt:

Tabelle V:

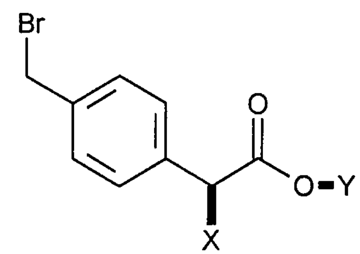

| Bsp.-Nr. | X | Y | a) Fp. (°C)<br>b) $R_f$ (Solvens) | Spektren | Ausgangsmaterial aus Bsp.-Nr. |
|---|---|---|---|---|---|
| XCV | H | Me | b) 0,45 (XA) | | LXIII |
| XCVI | H | tBu | b) 0,54 (V) | | LXV |
| XCVII | Me | tBu | b) 0,78 (I) | | LXVII |
| XCVIII | Et | tBu | b) 0,75 (I) | | LXVIII |
| XCIX | nPr | tBu | b) 0,80 (I) | | LXIX |
| C | iPr | Me | b) 0,78 (M) | | LXX |
| CI | iPr | tBu | b) 0,90 (Q) | | LXXI |
| CII | nBu | tBu | b) 0,82 (I) | | LXXII |
| CIII | iBu | tBu | b) 0,86 (M) | | LXXIII |
| CIV | nPent | tBu | b) 0,73 (H) | | LXXIV |
| CV | iPent | tBu | | MS: 372, 374 (($[M+NH_4]^+$; 79%, 77%) | LXXV |

| Bsp.-Nr. | —X | —Y | a) Fp. (°C)<br>b) $R_f$ (Solvens) | Spektren | Ausgangsmaterial aus Bsp.-Nr. |
|---|---|---|---|---|---|
| CVI | $—CH(CH_2CH_3)_2$ | tBu | | MS: 372, 372 ($[M+NH_4]^+$; 4%, 4%) | LXXVI |
| CVII | nHex | tBu | b) 0,85 (I) | | LXXVII |
| CVIII | $—CH_2CH(CH_2CH_3)_2$ | tBu | | MS: 386, 388 ($[M+NH_4]^+$; 14%, 14%) | LXXVIII |
| CIX | cPent | Me | b) 0,63 (Petrolether : Essigester = 10:1) | | LXXIX |
| CX | cHex | Me | b) 0,47 (Petrolether : Essigsäure = 20:1) | | LXXX |
| CXI | cHex | tBu | b) 0,58 (Petrolether : Essigsäure = 10:1) | | LXXXI |
| CXII | cHept | Me | b) 0,59 (I) | | LXXXII |
| CXIII | cHept | tBu | b) 0,84 (M) | | LXXXIII |
| CXIV | cOct | tBu | b) 0,49 (Petrolether : Essigester = 20:1) | | LXXXIV |
| CXV | 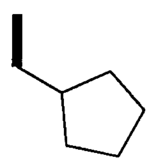 | tBu | b) 0,58 (A) | | LXXXV |

| Bsp.-Nr. | —X | —Y | a) Fp. (°C)<br>b) $R_f$ (Solvens) | Spektren | Ausgangsmaterial aus Bsp.-Nr. |
|---|---|---|---|---|---|
| CXVI | 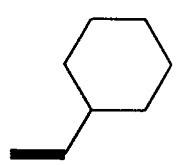 | tBu | $^1$H-NMR (250 MHz, $CDCl_3$, TMS): $\delta$ = 3,58 (M; 1H), 4.49 (s; 2H) ppm | | LXXXVI |

Die Verbindungen der Tabelle VI werden analog der Vorschrift von Beispiel XLIV hergestellt:

**Tabelle VI**

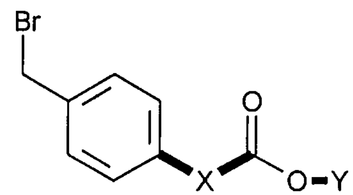

| Bsp.-Nr. | 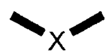 | 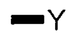 | a) Fp. (°C)<br>b) $R_f$ (Solvens) | Spektren | Ausgangsmaterial aus Bsp.-Nr. |
|---|---|---|---|---|---|
| CXVII | 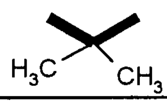 | tBu | b) 0,68 (F) | | LXXXVII |
| CXVIII | 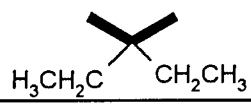 | tBu | b) 0,38 (Petrolether : Essisäure = 20:1) | | LXXXVIII |
| CXIX | 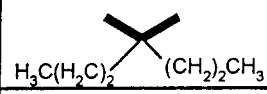 | tBu | b) 0,84 (B) | | LXXXIX |
| CXX | 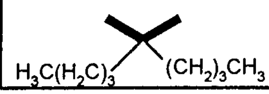 | tBu | b) 0,82 (C) | | XC |

| Bsp.-Nr. | 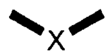 | 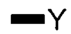 | a) Fp. (°C)<br>b) $R_f$ (Solvens) | Spektren | Ausgangsmaterial aus Bsp.-Nr. |
|---|---|---|---|---|---|
| CXXI | 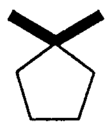 | tBu | | MS: 356, 358 ($[M+NH_4]^+$; 9%, 11%) | XCI |
| CXXII | 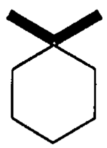 | tBu | | MS: 370, 372 ($[M+NH_4]^+$; 5%, 5%) | XCII |
| CXXIII | 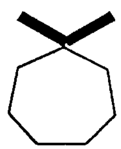 | tBu | b) 0,47 (Petrolether : Essigsäure = 20:1) | | XCIII |

**Beispiel CXXIV**

2(S)-2-Cyclopentyl-2[4-(2,4-dimethyl-α-carbolin-9-yl)methyl]phenyl-essigsäure-L-menthylester

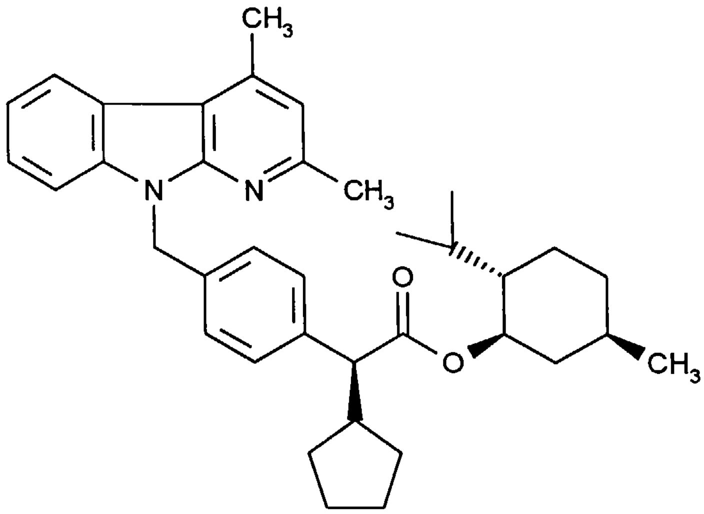

Die Reaktion wird unter Stickstoffatmosphäre durchgeführt. 480 g (2,44 mol) Carbolin werden in 4,13 l Dimethylformamid suspendiert und unter Rühren mit 287,7 g Kalium-tert.butylat gelöst in 1 l Dimethylformamid versetzt. Die Reaktionslösung erwärmt sich auf 30°C. Nach 30 min wird der Ansatz auf 20°C abgekühlt. Anschließend werden 1,707 kg (2,69 mol) 69%iges Menthylesterbromid (CXXXV), gelöst in 1,56 l Dimethylformamid, so zugetropft, daß die Innentemperatur nicht über 35°C ansteigt. Nach weiteren 15 min Reaktionszeit wird die Reaktionslösung in ein Gemisch aus 1,8 l 0%igeNatriumchloridlösung und 13 l Essigester gegossen. Nach 20 min unter Rühren wird die Essigesterphase abgetrennt und zweimal mit je 3 l 10%iger Natriumchloridlösung extrahiert. Nach dem Trocknen der organischen Phase über Natriumsulfat wird Essigester im Vakuum bei ca. 40°C abdestilliert. Der sirupöse Rückstand wird in 4,4 l Methanol aufgenommen und 30 min unter Rückfluß, 12 h bei Raumtemperatur gerührt. Die ausgefallenen Kristalle werden abgesaugt, mit Methanol gewaschen und im Vakuum bei 40°C getrocknet.

Ausbeute: 947 g (70,6% d.Th.)
Schmelzpunkt: 142°C

**Beispiel CXXV**

2-(S)-2-Cyclopentyl-2-[4-(2,4-dimethyl-α-carbolin-9-yl)-methyl]phenylessigsäure

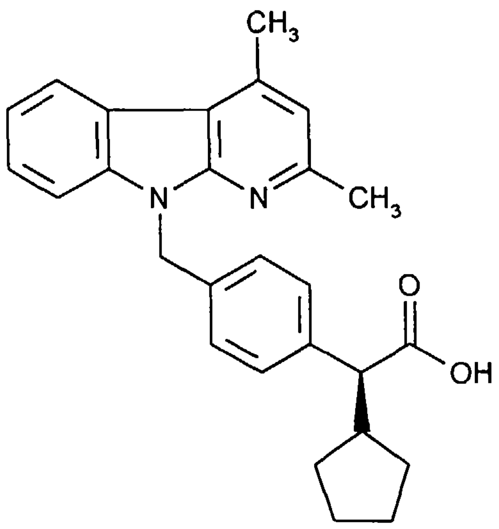

947 g (1,72 mol) der Verbindung aus Beispiel CXXIV werden mit 2,4 l Ameisensäure versetzt. Unter Rühren werden 1,21 l wäßrige Bromwasserstoffsäure (48%ig) zugetropft. Die erhaltene Suspension wird 6 Stunden bei 95-98°C gerührt und anschließend auf Raumtemperatur abgekühlt. Die Reaktionslösung wird unter Rühren mit 1,6 l Isopropanol und 3,2 l Wasser versetzt. Unter leichter Kühlung wird mit 45%iger Natronlauge ein pH-Wert von 5 eingestellt (Verbrauch an Natronlauge: 5,2 kg). Der Niederschlag wird abgesaugt, zweimal mit 5,7 l Wasser gewaschen und trocken gesaugt. Anschließend wird das wasserfeuchte Produkt in 2,6 l Isopropanol 2 Stunden bei Raumtemperatur ausgerührt. Das Kristallisat wird abgesaugt, mit 2,8 l Isopropanol nachgewaschen und im Vakuum bei 60°C getrocknet.

Ausbeute: 574 g (81% d.Th.)
Schmelzpunkt: 197-199°C

**Beispiel CXXVI**

2-(S)-2-Cyclopentyl-2-[4-(2,4-dimethyl-α-carbolin-9-yl)-methyl]phenylessigsäurechlorid

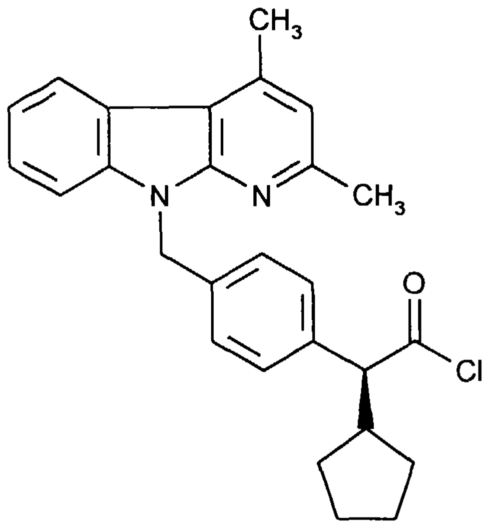

Eine Suspension von 350 g (0,85 mol) der Verbindung aus Beispiel CXXV in 3 l Methylenchlorid wird unter Rühren zum Rückfluß erhitzt. Innerhalb von 1 h werden 95 ml (155 g, 1,3 mol) Thionylchlorid zugetropft und weitere 2 h unter Rückflußtemperatur gerührt. Anschließend wird die Reaktionslösung auf Raumtemperatur abgekühlt, bei 25-30°C im Vakuum bis zur beginnenden Kristallisation eingeengt und mit 2,5 l Toluol versetzt. Bei einer Temperatur von 30-40°C werden im Vakuum weitere 2,3 l Lösemittel abdestilliert. Nach Abkühlen auf ca. 20°C werden 1,2 l Toluol zu dem Ansatz gegeben. Die Suspension wird auf 0-5°C abgekühlt, 1 h bei dieser Temperatur gerührt, abgesaugt und mit 1,4 l Toluol nachgewaschen und trocken gesaugt. Das toluolfeuchte Produkt wird ohne weitere Charakterisierung umgesetzt.

**Beispiel CXXVII**

2-Cyclopentyl-2-(3-tolyl)-essigsäuremethylester

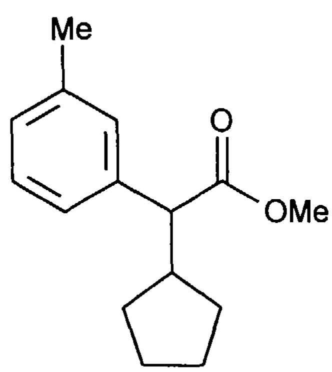

Die Titelverbindung wird analog der Vorschrift des Beispiels LXIII aus 2-(3-Tolyl)-essigsäuremethylester hergestellt.

$R_f$ = 0,56 (P)

**Beispiel CXXVIII**

2-(3 Brommethyl-phenyl)-2-cyclopentyl-essigsäuremethylester

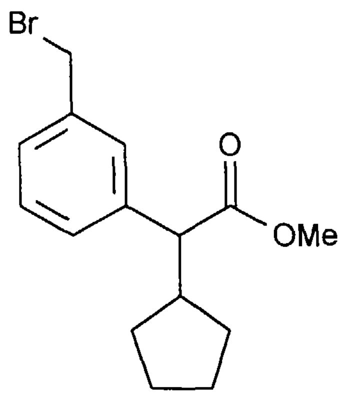

Die Titelverbindung wird analog der Vorschrift für Beispiel XCIV aus der Verbindung des Beispiels CXXVII hergestellt.

$R_f$ = 0,40 (P)

**Beispiel CXXIX**

2(R/S)-2-Cyclopentyl-2-(4-methylphenyl)-essigsäure

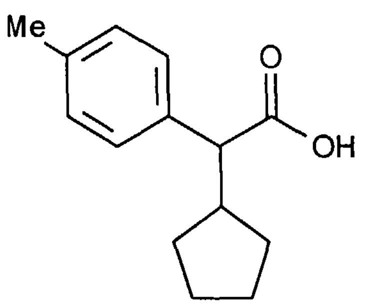

In einem 40 l-Rührwerkkessel mit angeschlossenem Waschturm werden 2,0 kg (7,2 mol) der Verbindung aus Beispiel LXV in 4 l Dioxan gelöst. Nach Zugabe Von 4,5 l konzentrierter Salzsäure wird bis zum vollständigen Umsatz (3h) bei 50°C gerührt. Das Reaktionsgemisch wird mit Eis versetzt und mit konzentrierter Natronlauge auf pH = 12 eingestellt. Nach Zugabe von Wasser bis zur vollständigen Auflösung der Feststoffe wird mit Essigsäure gewaschen, die organische Phase wird mit verdünnter Natronlauge gewaschen und die Vereinigten wäßrigen Phasen unter Kühlung mit konzentrierter Salzsäure auf pH = 1 eingestellt. Es wird zweimal mit Essigester gewaschen, über Natriumsulfat getrocknet und eingeengt.

Ausbeute: 1,27 kg; 81% d.Th.
Schmpkt.: 92°C
$R_f$ = 0,20 (Petrolether : Essigester = 4:1)
$^1$H-NMR ($CDCl_3$, 200 MHz, TMS): $\delta$ = 0,98 (m, 1H); 1,20 - 1,71 (m, 6H); 1,82 - 2,05 (m, 1H); 2,31 (s, 3H); 2,52 (m, 1H); 3,21 (d, 1H); 7,10 (m, 2H); 7,21 m, 2H); 11,90 (br, s, 1H) ppm.

**Beispiel CXXX**

(S)-(+)-2-Cyclopentyl-2-(4-methylphenyl)-essigsäure

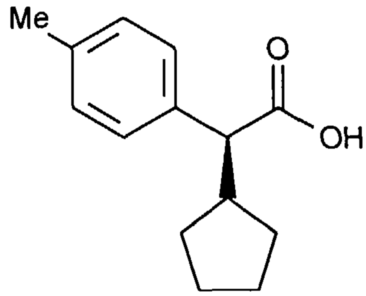

Zu einer Suspension von 560 g (2,57 mol) der Verbindung aus Beispiel CXXIX in 4,8 l Wasser werden unter Rühren 2,4 l THF und 129,7 g (1,28 mol) Triethylamin gegeben. Die entstehende Lösung wird auf 60°C erwärmt, es werden 155,4 g (1,28 mmol) (S)-(-)-Phenethylamin zugegeben und die anfallende Suspension 2 h bei 60°C gerührt. Das Reaktionsgemisch wird auf 20°C gekühlt, der Niederschlag wird abgesaugt, mit 2,4 l Wasser / THF (2:1) gewaschen und im Vakuum getrocknet.

Ausbeute: 360 g Phenethylammoniumsalz; 41,3% d.Th. bezogen auf Racemat Bsp.-Nr. CXXIX
In 3 l Wasser werden 745 g (2,2 mol) Phenethylammoniumsalz suspendiert, mit verdünnter Salzsäure (1:1) angesäuert (pH = 1) und 30 Minuten gerührt. Die ölige Suspension wird dreimal mit je 1 l Dichlormethan gewaschen, die vereinigten organischen Phasen mit Wasser gewaschen, über Natriumsulfat getrocknet und eingeengt, wobei der Rückstand kristallisiert.
Ausbeute: 475 g; 37,3% d.Th. bezogen auf Racemat Bsp.-Nr. CXXIX ee: 96,3% (HPLC)
Schmpkt.: 66°C
Durch Kristallisation des Phenethylammoniumsalzes aus THF und Freisetzung von Beispiel Nr. CXXX, wie oben beschrieben wird das Reinenantiomer erhalten: ee: >99,5% (HPLC)
Drehwert: $[\alpha]^{20}_{D}$ = +59,55 (Ethanol / c = 0,85)
Die HPLC-Methode zur Bestimmung des eeWertes ist folgende (die racemische Verbindung aus Beispiel CXXIX dient als Vergleich):

| | |
|---|---|
| Säule: | Chiracel OJ (Daicel) |
| Partikelgröße: | 10 μ |
| Packing: | 250 x 2 mm (Fa. Grom) |
| Mobile Phase: | n-Heptan: 2-Propanol = 97:3 |
| Fluß: | 0,2 ml/min |
| Eingangsdruck: | 22 bar |

**Beispiel CXXXI**

(S)-(+)-2-Cyclopentyl-2-(4-methylphenyl)essigsäure-tert.butylester

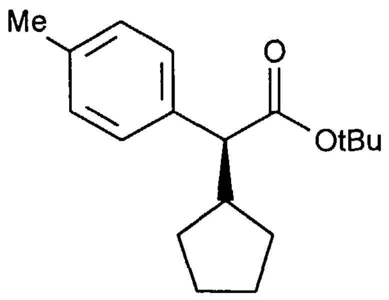

Zu einer Lösung von 465 g (2,13 mol) der Verbindung aus Beispiel CXXX in 1,4 l Dichlormethan werden 6 ml konzentrierte Schwefelsäure gegeben, wobei sich eine Temperatur Von ca. 10°C einstellt. In ein Dewargefäß werden 550 ml (5 mol) Isobuten einkondensiert und in einer Portion zur Eduktlösung gegeben. Das Reaktionsgemisch wird über Nacht gerührt. Zur Vervollständigung des Umsatzes werden nochmals 6 ml konzentrierte Schwefelsäure und 500 ml Isobuten zugegeben und über Nacht gerührt. Nach Zugabe von 40 g Kaliumcarbonat wird 3 h gerührt, und darauf 2 l Wasser zugegeben, wobei es anfangs zu einer starken Gasentwicklung kommt. Es wird dreimal mit je 2 l Dichlormethan gewaschen, die vereinigten organischen Phasen werden mit 5 l Natriumchloridlösung gewaschen, über Natriumsulfat getrocknet und zu einem Öl, das langsam durchkristallisiert, eingeengt.

Ausbeute: 480 g; 82% d.Th.
Schmpkt.: 45°C
$R_f$ = 0,90 (Toluol : Essigester = 8:2)

**Beispiel CXXXII**

(S)-(+)-2-(4-Brommethylphenyl)-2-cyclopentyl-essigsäure-tert.butylester

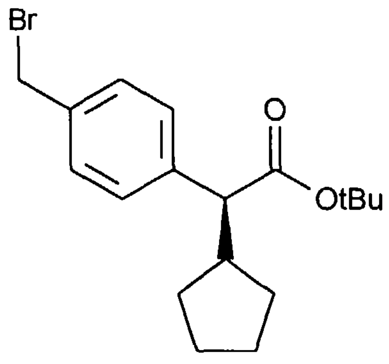

In einem 10 l-Kolben werden 480 g (1,75 mol) der Verbindung aus Beispiel CXXXI in 3,4 l Tetrachlormethan unter Rückfluß gelöst und mit 70 g Gesamtmenge von 311 g (1,75 mol) NBS sowie 14 g (0,085 mol) AIBN versetzt. Die Reaktion setzt nach ca 1 h Refluxieren ein; nach Abklingen wird weiteres NBS in 50 g Portionen zugegeben. Nach 5 h Refluxieren und anschließendem Stehen bei Raumtemperatur über Nacht wird zur Aufarbeitung auf 0°C gekühlt, das Succinimid abgesaugt und mit 600 ml Tetrachlormethan nachgewaschen. Die vereinigten Filtrate werden eingeengt

und Restlösemittel bis zur Gewichtskonstanz im Vakuum entfernt.

Rohausbeute: 570 g; ca. 100% d.Th.
HPLC: 68,8% (15,5% Edukt, 10,1% Dibromverbindung)

Der Reinstoff wird durch Säulenchromatographie erhalten

$R_f$ = 0,42 (Q)
$^1$H-NMR ($CDCl_3$, 200 MHz, TMS): $\delta$ = 0,98 (m, 1H); 1,22 - 1,71 (m, 6H); 1,40 (s, 9H); 1,90 (m, 1H); 2,47 (m, 1H); 3,16 (d, 1H); 4,49 (s, 2H); 7,32 (m, 4H) ppm.

**Beispiel CXXXIII**

2-(4-Tolyl)-essigsäure-(L)-menthylester

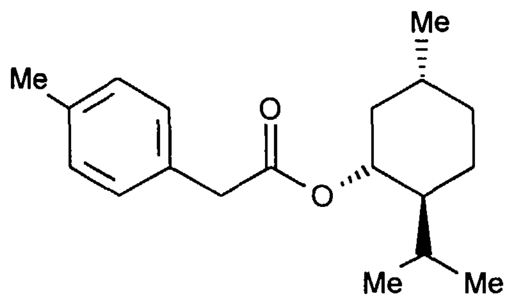

3,15 kg p-Tolylessigsäure und 9,45 l Toluol werden vorgelegt. Unter Rühren und Kühlen werden 3,115 kg L-Menthol und 21,4 ml Methansulfonsäure zugegeben. Anschließend wird auf Rückflußtemperatur erhitzt und innerhalb von 16 bis 20 Stunden über einen Wasserabscheider die entsprechende Menge Wasser abgetrennt. Nach Abkühlen auf Raumtemperatur wird einmal mit 4,41 l gesättigter Natriumhydrogencarbonat-Lösung und zweimal mit je 4,41 l Wasser ausgerührt. Die organische Phase wird vom Lösemittel befreit und ergibt 5,725 kg gewünschte Verbindung (GC 99,9%, Retentionszeit 19,49 min).

$^1$H-NMR ($CDCl_3$, ppm): 7,05 - 7,15 (4H, m); 4,55 (1H, txd); 3,5 (2H, s); 2,8 (3H, s)); 0,65 (3H, s).

**Beispiel CXXXIV**

2-(S)-2-Cyclopentyl-2-(4-tolyl)-essigsäure-(L)-menthylester

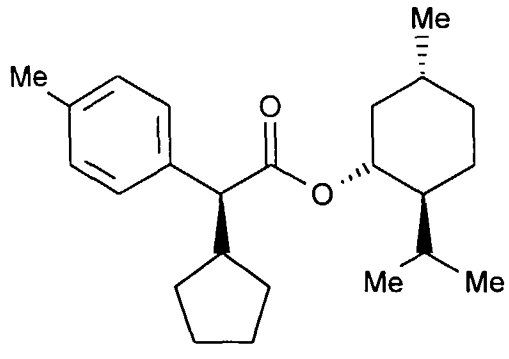

1,575 kg Kalium-tert.butanolat werden bei Raumtemperatur in 3,75 l DMF gelöst. Man kühlt auf 10°C ab und läßt innerhalb von 45 Minuten bei dieser Temperatur 2,678 kg der Verbindung aus Beispiel CXXXIII zulaufen und spült mit 0,375 l DMF nach. Innerhalb von 1 bis 2 Stunden werden nun unter voller Kühlung 1,658 kg Cyclopentylbromid zugepumpt. Die Suspension wird ohne Kühlung noch eine Stunde nachgerührt und dann auf -7°C abgekühlt. Bei Erreichen von - 10°C wird mit dem richtigen Diastereomeren angeimpft und dann weiter auf -7°C gekühlt. Nach Erreichen von -7°C wird 3 bis 4 Stunden bei dieser Temperatur nachgerührt. Die Aufarbeitung erfolgt durch Einbringen der Reaktionssuspension in ein Gemisch aus 1,5 kg Eis und 6 kg Wasser. Der Ansatz wird danach bei 0 bis 2°C über Nacht gerührt. Die Aufarbeitung erfolgt durch Absaugen der Suspension und Waschen der Kristalle mit insgesamt 2,5 l Wasser. Die Kristalle werden bei 45°C im Vakuumtrockenschrank getrocknet. Es werden 3,289 kg eine 85 zu 15 Diastereomerengemisches erhalten.
4,345 kg einer wie oben beschrieben, hergestellten Mischung, werden in 21,75 l bei 30 bis 35°C gelöst. Nach Animpfen mit dem richtigen Diastereomeren und Abkühlen auf Raumtemperatur wird über Nacht gerührt und am nächsten Morgen auf 0 bis 5°C gekühlt. Nach 1 bis 2 Stunden bei dieser Temperatur werden die Kristalle abgesaugt, getrocknet oder erneut umkristallisiert. Durch ein bis zweimaliges Wiederholen der Methanolkristallisation kann Material mit einer Diastereomerenreinheit von ≥ 99,5% hergestellt werden (GC-Retentionszeit 22,61 min).
Die Ausbeute an diastereomerenreiner Titelverbindung liegt bei 65-70% über die Stufen Cyclopentylierung und Reinkristallisation und kann durch Rekristallisation bzw. durch Epimerisierung der Mutterlaugen mit Kalium-tert.butanolat in DMF und erneute Kristallisation des rohen Diastereomerengemisches auf 75-80% gesteigert werden.

$^{13}C$-NMR ($CDCl_3$, CH-Signale, ppm) 128,90; 128,92; 73,96; 57,85; 46,92; 43,13; 31,28; 25,96.

**Beispiel CXXXV**

2-(S)-2-(4-Brommethyl-phenyl)-2-cyclopentyl-essigsäure-(L)-menthylester

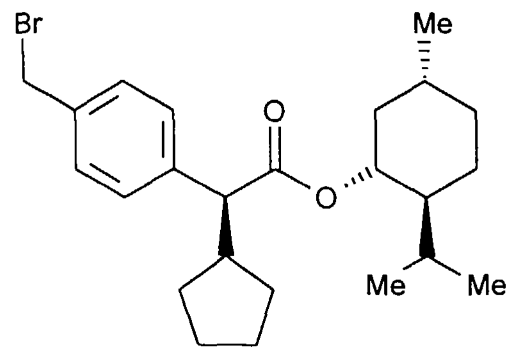

1,40 kg der Verbindung aus Beispiel CXXXIV werden in 13,74 l Chlorbenzol auf 80°C erwärmt. Danach gibt man 0,618 kg 1,3-Dibrom-5,5-dimethylhydantoin hinzu und erwärmt weiter auf 85°C. Bei dieser Temperatur wird nun zum Starten der Reaktion 20,4 g AIBN gegeben. Die Temperatur steigt nach Beginn der Reaktion auf 90 bis 105°C, sinkt dann aber wieder auf etwa 85°C ab. Insgesamt wird 2 Stunden nachreagiert. Danach wird der Kesselinhalt auf Raumtemperatur abgekühlt und eine Stunde nachgerührt. Es wird von den ausgefallenen Kristallen abgesaugt und das Filtrat vom Lösemittel befreit. Das zurückbleibende Öl ist nach HPLC-Analyse (Retentionszeit 14,68 min.) 61,2%ig. Es werden 1,69 kg erhalten. Das Gemisch kann roh in die folgenden Alkylierungen eingesetzt werden. Chromatographie und nachfolgende Kristallisation liefern ein weißes Pulver vom Schmelzpunkt 57-58°C, mit korrekter CH-Analyse.

$^{1}H$-NMR ($CDCl_3$, ppm): 7,3 (4H, s); 4,65 (1H, txd); 4,45 (2H, s); 3,35 (1H, d); 0,65 (3H, d).

**Beispiel CXXXVI**

2-(R/S)-2-Phenyl-2-(4-methyl)phenylessigsäuremethylester

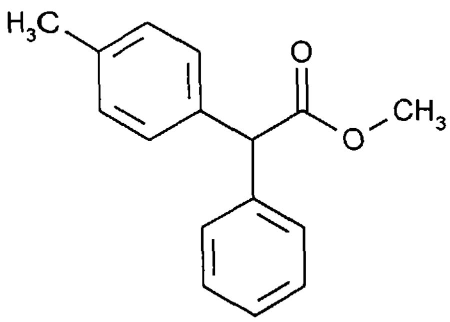

21,0 g (100 mmol) 2-Phenyl-1-(4-methyl)phenyl-1-oxoethan und 38,8 g (120 mmol) Iodbenzoldiacetat werden in 300 ml Orthoameisensäuretrimethylester gelöst. Zu dieser Lösung werden 19,6 g konzentrierte Schwefelsäure gegeben, und die Lösung wird für 6 h bei 60°C gerührt. Die Lösung wird auf Raumtemperatur abgekühlt, mit Wasser vedünnt, und mit Diethylether extrahiert. Die vereinigten organischen Phasen werden über Natriumsulfat getrocknet und einrotiert. Der Rückstand wird säulenchromatographisch gereinigt.

Ausbeute: 13,1 g (55%)
$R_f$ = 0,33 (Q)
MS (FAB): 241 (25%), 181 (100%).
$^1$H-NMR (200 MHz, $CDCl_3$, TMS): $\delta$ = 7,3 - 7,10 (m, 9H); 4,99 (s, 1H); 3,73 (s, 3H); 2,31 (s, 3H) ppm.

**Beispiel CXXXVII**

2-(R/S)-2-(4-Chlorphenyl)-2-(4-tolyl)essigsäure-methylester

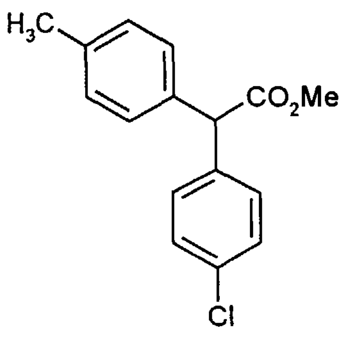

In Analogie zur Vorschrift des Beispiels CXXXVI wird die Titelverbindung hergestellt.

**Herstellungbeispiele**

**Beispiel 1**

2-(S)-2-Cyclopentyl-2-[4-(2,4-dimethyl-α-carbolin-9-yl)-methyl]phenylessigsäure-N-propylamid

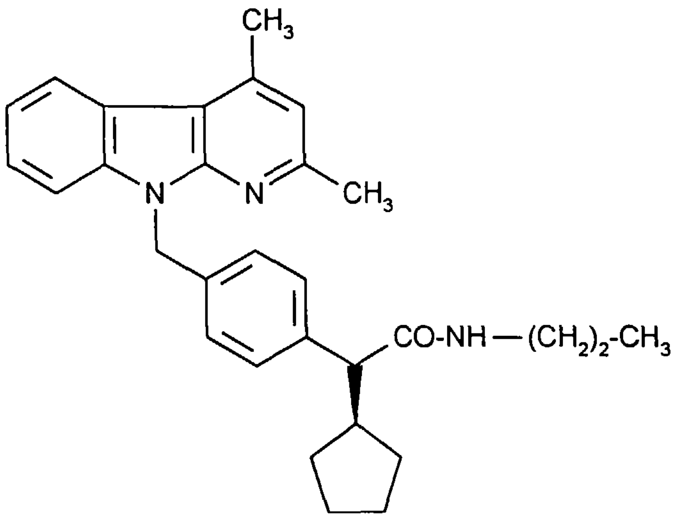

200 mg (0,46 mmol) Tolylessigsäurechlorid (CXXVI) werden in 2 ml Methylenchlorid gelöst, 28 mg (0,46 mmol) Propylamin und 0,1 ml (0,70 mmol) Triethylamin zugegeben und 45 min bei Raumtemperatur gerührt. Dann wird mit 1 ml Wasser versetzt, 5 min bei Raumtemperatur gerührt und auf eine Glas-Fertigsäule (Extrelut3, Fa. E. Merck) gegeben. Nach 5 min wird mit 15 ml Methylenchlorid eluiert, das Filtrat eingeengt und im Vakuum getrocknet.

Ausbeute: 177 mg (85% d.Th.)
$R_f$ = 0,74 (A)

In Analogie zur Vorschrift des Beispiels 1 werden die in der Tabelle 1 aufgeführten Verbindungen hergestellt:

**Tabelle 1:**

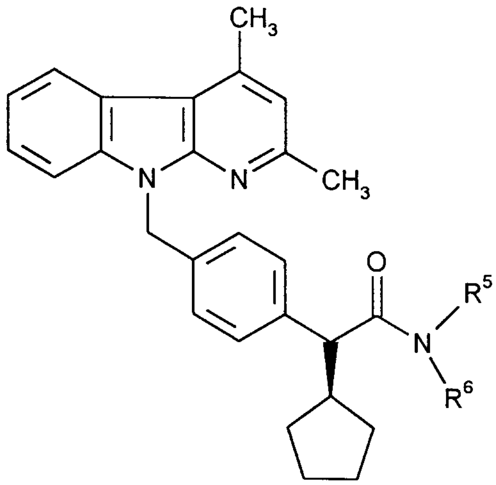

| Bsp.-Nr. | $R^5$ | $R^6$ | Ausbeute (% d.Th.) | $R_f$ / Fp. (°C) |
|---|---|---|---|---|
| 2 | Ph | H | 90 | 0,85 (A) |
| 3 | $-CH_2-CH_2OCH_2CH_2-$ | | 96 | 0,70 (A) |
| 4 | 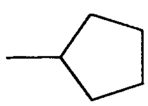 | H | 89 | 0,75 (A) |
| 5 | 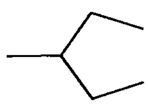 | H | 79 | 0,70 (A) |
| 6 | $-(CH_2)_7-$ | | 91 | 0,84 (A) |
| 7 | Me | H | 90 | 0,73 (A) |
| 8 | Et | H | 69 | 201°C |
| 9 | $-(CH_2)_3-Ph$ | H | 89 | 118°C |
| 10 | n-Oct | n-Oct | 92 | 0,95 (A) |
| 11 | 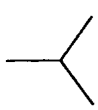 | H | 35 | 235°C |
| 12 | n-Hex | n-Hex | 53 | 0,93 (A) |
| 13 | 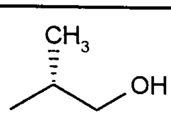 | H | 64 | 234°C |
| 14 | 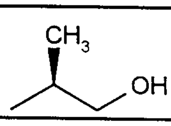 | H | 69 | 232°C |

| Bsp.-Nr. | $R^5$ | $R^6$ | Ausbeute (% d.Th.) | $R_f$ / Fp. (°C) |
|---|---|---|---|---|
| 15 | 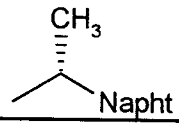 | H | 79 | 240°C |
| 16 | 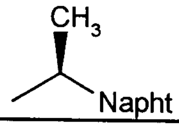 | H | 73 | 248°C |
| 17 | $-CH_2CF_3$ | H | 62 | 216°C |
| 18 | 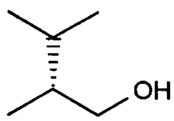 | H | 82 | 185°C |
| 19 | $-CH_2CH_2OH$ | H | 61 | 198°C |
| 20 | n-Pent | H | 88 | 178°C |
| 21 | n-Hex | H | 88 | 178°C |
| 22 | n-Oct | H | 90 | 0,89 (A) |
| 23 | n-Pr | H | 84 | 0,74 (A) |
| 24 | 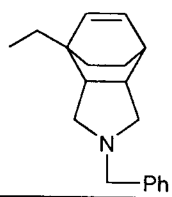 | H | 24 | 0,58 (W) |
| 25 | 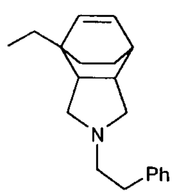 | H | 40 | 0,52 (W) |
| 26 | 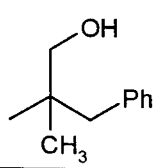 | H | 35 | 0,74 (W) |
| 27 | 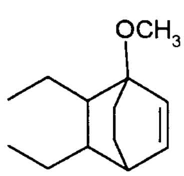 | | 37 | 0,80 (W) |

**Beispiel 28**

2-(S)-2-Cyclopentyl-2-[4-(2,4-dimethyl-α-carbolin-9-yl)-methylphenyl]essigsäure-N-(1-phenyl-ethen-1-yl)amid

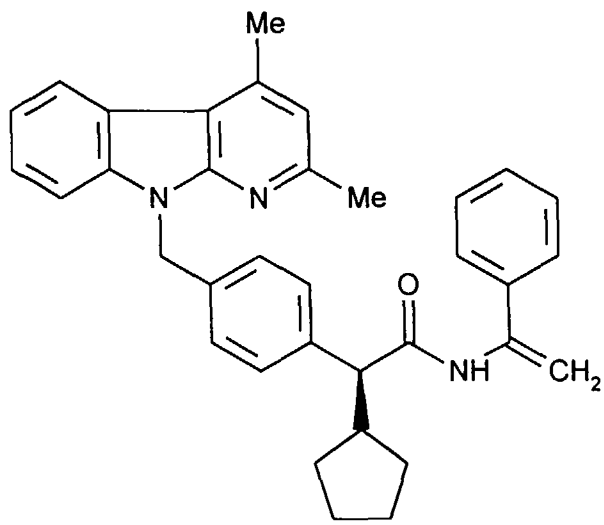

2,00 g (3,76 mmol) 2-(S)-2-Cyclopentyl-2-[4-(2,4-dimethyl-α-carbolin-9-yl)methylphenyl]-essigsäure-N-((1R)-1-phenyl-2-hydroxy)-ethanamid werden in 20 ml wasserfreiem DMF gelöst, mit 0,52 ml (3,76 mmol) Triethylamin versetzt und bei -30°C mit Mesylchlorid verrührt. Nach 2 Stunden wird auf 20°C aufgewärmt, mit weiteren 1,04 ml (7,52 mmol) Triethylamin versetzt und 20 Stunden nachgerührt. Das Reaktionsgemisch wird mit Diethylether und einem wäßrigen Puffer von pH = 2 (Merck) verdünnt, die Phasen getrennt und die organische Phase im Vakuum eingedampft. Der Rückstand wird in Methanol umkristallisiert, nach dem Abkühlen abgesaugt, mit kaltem Methanol nachgewaschen und im Vakuum über Phosphorpentoxid getrocknet.

Ausbeute: 0,81 g (42% d.Th.)
$R_f$ = 0,60 (G)
$^1$H-NMR ($d_6$-DMSO, 200 MHz, TMS): δ = 3,84 (dd, 1H); 4,53 (dd, 1H); 5,08 (dd, 1H) ppm.
MS (ES): m/z = 514 ($[M+H]^+$, 100%).

**Beispiel 29**

2-(S)-2-Cyclohexyl-2-[4-{(2,4-dimethyl-α-carbolin-9-yl)-methyl}phenyl]essigsäure-N-(1-phenyl-ethen-1-yl)amid

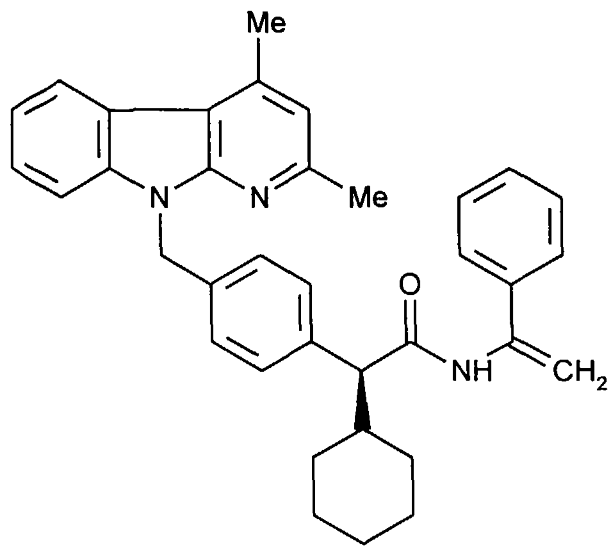

In Analogie zur Vorschrift des Beispiels 24 wird die Titelverbindung aus 2-(S)-2-Cyclohexyl-2-[4-(2,4-dimethyl-α-carbolin-9-yl)methylphenyl]-essigsäure-N-((1R)-1-phenyl-2-hydroxy)-ethanamid hergestellt

$R_f$ = 0,63 (G)
MS (ES): m/z = 528 ($[M+H]^+$, 100%).

**Patentansprüche**

1. Neue Arylessigsäureamide der allgemeinen Formel (I)

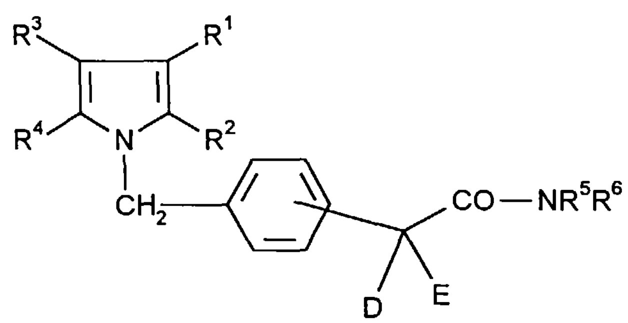

(I),

in welcher

$R^1$ und $R^2$ unter Einbezug der sie verbindenden Doppelbindung gemeinsam einen Phenyl- oder Pyridylring oder einen Ring der Formel

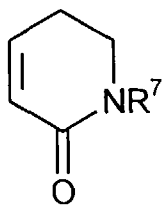

bilden,
worin

| | |
|---|---|
| $R^7$ | Wasserstoff oder geradkettiges oder verzweigtes Alkyl mit bis zu 4 Kohlenstoffatomen bedeutet, |
| $R^3$ und $R^4$ | unter Einbezug der sie verbindenden Doppelbindung gemeinsam einen Phenylring oder einen 4- bis 8-gliedrigen Cycloalken-oder Oxocycloalken-Rest bilden, wobei alle unter $R^1/R^2$ und $R^3/R^4$ aufgeführten Ringsysteme gegebenenfalls bis zu 3-fach gleich oder verschieden durch Halogen, Trifluormethyl, Carboxy, Hydroxy, durch geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert sind, das seinerseits durch Hydroxy oder durch geradkettiges oder verzweigtes Alkoxy mit bis zu 4 Kohlenstoffatomen substituiert sein kann, |
| D und E | gleich oder verschieden sind und für Wasserstoff, Cycloalkyl mit 3 bis 8 Kohlenstoffatomen oder geradkettiges oder verzweigtes Alkyl mit bis zu 10 Kohlenstoffatomen stehen, das gegebenenfalls durch Cycloalkyl mit 3 bis 6 Kohlenstoffatomen substituiert ist, oder für Phenyl stehen, das gegebenenfalls durch Halogen oder Trifluormethyl substituiert ist, oder |
| D und E | gemeinsam unter Einbezug der CH-Gruppe einen 4- bis 8-gliedrigen Carbocyclus bilden, |
| $R^5$ | für Wasserstoff, geradkettiges oder verzweigtes Alkyl mit bis zu 12 Kohlentsoffatomen oder für Cycloalkyl mit 3 bis 8 Kohlenstoffatomen steht, |
| $R^6$ | für Cycloalkyl mit 3 bis 8 Kohlenstoffatomen oder Phenyl steht, oder für geradkettiges oder verzweigtes Alkyl mit bis zu 9 Kohlenstoffatomen steht, das gegebenenfalls durch Hydroxy, Naphthyl, Trifluormethyl oder durch einen Rest der Formel |

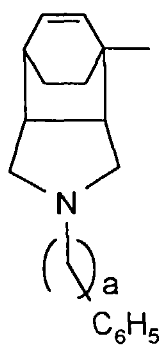

substituiert ist,
worin

| | |
|---|---|
| a | eine Zahl 1 oder 2 bedeutet oder |
| $R^6$ | für einen Rest der Formel $-(CH_2)_n-R^8$ steht, worin |
| n | eine Zahl 2, 3, 4 oder 5 bedeutet, |
| $R^8$ | Naphthyl oder Phenyl bedeutet, die gegebenenfalls durch Carboxyl, Trifluormethyl, Halogen, Hydroxy, Trifluormethoxy oder durch geradkettiges oder verzweigtes Alkyl, Acyl, Alkoxy oder Alkoxycarbonyl mit jeweils bis zu 6 Kohlenstoffatomen substituiert sind, oder für einen Rest der Formel |

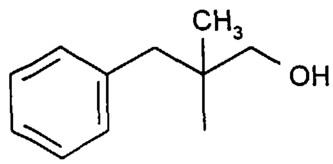 oder 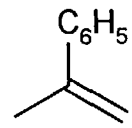 steht,

oder

R⁵ und R⁶ gemeinsam mit dem Stickstoffatom einen heterocyclischen Rest der Formel $-(CH_2)_2-O-(CH_2)_2$, $-CH_2-(CH_2)_p-CH_2-$,

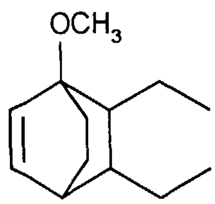 oder 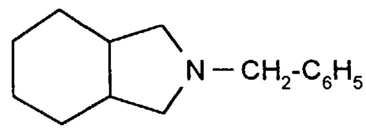

bilden

worin

p eine Zahl 2, 3, 4, 5, 6, 7, 8 oder 9 bedeutet,

gegebenenfalls in einer isomeren Form und deren Salze.

**2.** Neue Arylessigsäureamide der Formel nach Anspruch 1 in welcher

$R^1$ und $R^2$ unter Einbezug der sie verbindenden Doppelbindung gemeinsam einen Phenyl- oder Pyridylring oder einen Ring der Formel

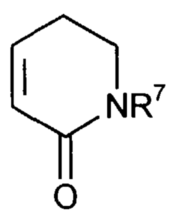

bilden,

worin

$R^7$ Wasserstoff oder geradkettiges oder verzweigtes Alkyl mit bis zu 3 Kohlenstoffatomen bedeutet,

$R^3$ und $R^4$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten-oder Oxocycloocten-Rest bilden,

wobei alle unter $R^1$/$R^2$ und $R^3$/$R^4$ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 4 Kohlenstoffatomen oder durch geradkettiges oder verzweigtes Alkyl mit bis zu 4 Kohlenstoffatomen substituiert sind, das seinerseits durch Hydroxy oder durch geradkettiges oder verzweigtes Alkoxy mit bis zu 3 Kohlenstoffatomen substituiert sein kann,

D und E gleich oder verschieden sind und

für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 8 Kohlenstoffatomen stehen, das gegebenenfalls

durch Cyclopropyl, Cylcopentyl oder Cyclohexyl substituiert ist, oder
für Phenyl stehen, das gegebenenfalls durch Fluor, Chlor oder Brom substituiert ist,
oder

D und E gemeinsam unter Einbezug der CH-Gruppe einen Cyclobutyl-, Cyclopentyl-, Cyclohexyl-, Cycloheptyl- oder Cyclooctylring bilden,

$R^5$ für Wasserstoff, geradkettiges oder verzweigtes Alkyl mit bis zu 10 Kohlenstoffatomen, Cyclopropyl, Cyclobutyl, Cyclopentyl, Cyclohexyl, Cycloheptyl oder Cyclooctyl steht,

$R^6$ für Cyclopropyl, Cyclobutyl, Cyclopentyl, Cyclohexyl, Cycloheptyl, Cyclooctyl oder Phenyl steht, oder für geradkettiges oder verzweigtes Alkyl mit bis zu 7 Kohlenstoffatomen steht, das gegebenenfalls durch Hydroxy, Naphthyl, Trifluormethyl oder durch einen Rest der Formel

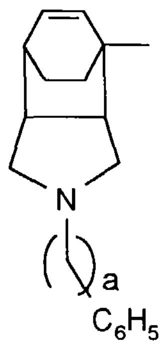

worin

a eine Zahl 1 oder 2 bedeutet,

substituiert ist,
oder

$R^6$ für einen Rest der Formel $-(CH_2)_n-R^8$ steht,
worin

n eine Zahl, 2, 3 oder 4 bedeutet,

$R^8$ Naphthyl oder Phenyl bedeutet, die gegebenenfalls durch Trifluormethyl, Fluor, Chlor, Brom, Hydroxy, Trifluormethoxy oder durch geradkettiges oder verzweigtes Alkyl, Alkoxy oder Alkoxycarbonyl mit jeweils bis zu 5 Kohlenstoffatomen substituiert sind,
oder
für einen Rest der Formel

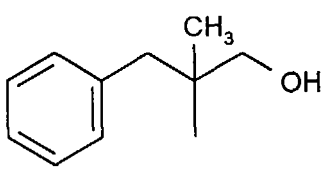 oder 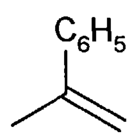 steht,

oder

$R^5$ und $R^6$ gemeinsam mit dem Stickstoffatom einen heterocyclischen Rest der Formel $-(CH_2)_2-O-(CH_2)_2$, $-CH_2-(CH_2)_p-CH_2-$,

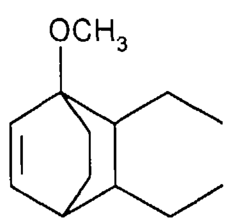 oder 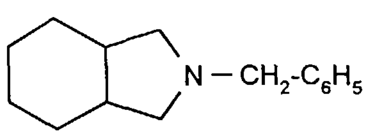

bilden,
worin

p eine Zahl 2, 3, 4, 5, 6, 7 oder 8 bedeutet,

gegebenenfalls in einer isomeren Form und deren Salze.

**3.** Neue Arylessigsäureamide der Formel nach Anspruch 1 in welcher

$R^1$ und $R^2$ unter Einbezug der sie verbindenden Doppelbindung gemeinsam einen Phenyl- oder Pyridylring oder einen Ring der Formel

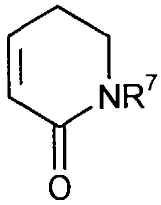

bilden,
worin

$R^7$ Wasserstoff oder Methyl bedeutet,

$R^3$ und $R^4$ unter Einbezug der sie verbindenden Doppelbindung gemeinsam einen Phenylring oder einen Cyclopenten-, Cyclohexen-, Cyclohepten-, Cycloocten-, Oxocyclopenten-, Oxocyclohexen-, Oxocyclohepten-oder Oxocycloocten-Rest bilden,
wobei alle unter $R^1$/$R^2$ und $R^3$/$R^4$ aufgeführten Ringsysteme gegebenenfalls bis zu 2-fach gleich oder verschieden durch Fluor, Chlor, Brom, Trifluormethyl, Carboxy, Hydroxy, durch geradkettiges oder verzweigtes Alkoxy oder Alkoxycarbonyl mit jeweils bis zu 3 Kohlenstoffatomen oder durch geradkettiges oder verzweigtes Alkyl mit bis zu 3 Kohlenstoffatomen substituiert sind, das seinerseits durch Hydroxy, Methoxy oder Ethoxy substituiert sein kann,

D und E gleich oder verschieden sind und
für Wasserstoff, Cyclopropyl, Cyclobutyl, Cyclopentyl, Cyclohexyl, Cycloheptyl, Cyclooctyl oder für geradkettiges oder verzweigtes Alkyl mit bis zu 6 Kohlenstoffatomen stehen, das gegebenenfalls durch Cyclopentyl oder Cyclohexyl substituiert ist, oder
für Phenyl stehen, das gegebenenfalls durch Fluor, Chlor oder Brom substituiert ist,
oder

D und E gemeinsam unter Einbezug der CH-Gruppe einen Cyclopentyl-, Cyclohexyl- oder Cycloheptylring bilden,

$R^5$ für Wasserstoff, geradkettiges oder verzweigtes Alkyl mit bis zu 8 Kohlenstoffatomen, Cyclopentyl, Cyclohexyl oder Cycloheptyl steht,

$R^6$ für Cylcopentyl, Cyclooctyl oder Phenyl steht, oder
für geradkettiges oder verzweigtes Alkyl mit bis zu 6 Kohlenstoffatomen steht, das gegebenenfalls durch Hydroxy, Naphthyl, Trifluormethyl oder durch einen Rest der Formel

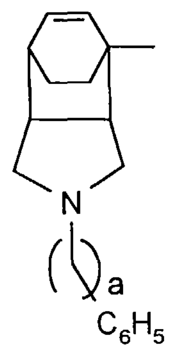

substituiert ist,
worin

a eine Zahl 1 oder 2 bedeutet,
oder

$R^6$ für einen Rest der Formel $-(CH_2)_n-R^8$ steht,
worin

n eine Zahl 2 oder 3 bedeutet,

$R^8$ Naphthyl oder Phenyl bedeutet, die gegebenenfalls durch Trifluormethyl, Fluor, Chlor, Hydroxy, Trifluormethoxy oder durch geradkettiges oder verzweigtes Alkyl, Alkoxy oder Alkoxycarbonyl mit jeweils bis zu 3 Kohlenstoffatomen substituiert sind,
oder
für einen Rest der Formel

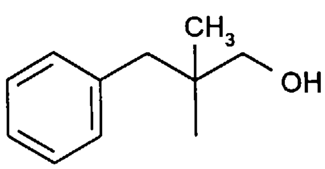 oder 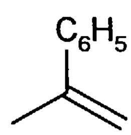 steht,

oder

$R^5$ und $R^6$ gemeinsam mit dem Stickstoffatom einen heterocyclischen Rest der Formel $-(CH_2)_2-O-(CH_2)_2$, $-CH_2-(CH_2)_p-CH_2-$,

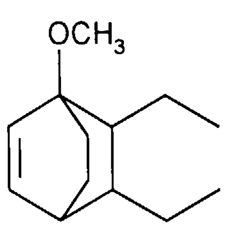 oder 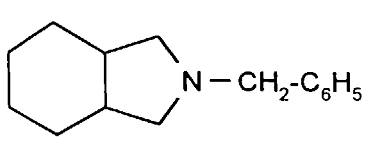

bilden,
worin

p eine Zahl 2, 3, 4, 5, 6 oder 7 bedeutet,

gegebenenfalls in einer isomeren Form und deren Salze.

**4.** Neue Arylessigsäureamide nach Anspruch 1 bis 3 als Arzneimittel.

**5.** Verfahren zur Herstellung von neuen Arylessigsäureamiden nach Anspruch 1 bis 3, dadurch gekennzeichnet, daß

man ausgehend von den (racemischen oder enantiomerenreinen) Carbonsäuren der allgemeinen Formel (II)

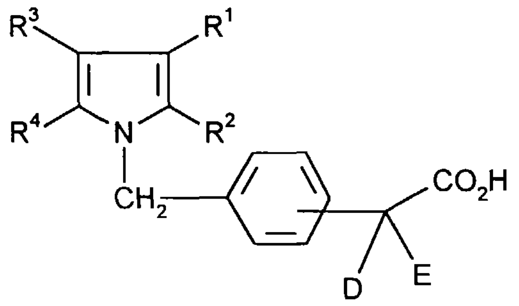

(II)

in welcher

D, E, $R^1$, $R^2$, $R^3$ und $R^4$ die angegebene Bedeutung haben,

zunächst die entsprechenden (racemischen oder enantiomerenreinen) Säurechloride der allgemeinen Formel (III)

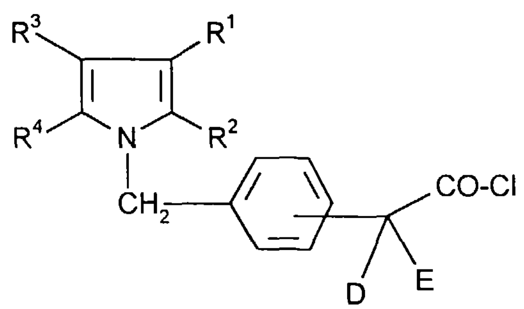

(III)

in welcher

D, E, $R^1$, $R^2$, $R^3$ und $R^4$ die angegebene Bedeutung haben,

herstellt und abschließend mit Aminen der allgemeinen Formel (IV)

$$HNR^5R^6 \quad (IV)$$

in welcher

$R^5$ und $R^6$ die angegebene Bedeutung haben,

in inerten Lösemitteln, gegebenenfalls in Anwesenheit von Basen und/oder Hilfsmitteln umsetzt.

**6.** Arzneimittel enthaltend mindestens ein neues Arylessigsäureamid nach Anspruch 1 bis 3 sowie pharmakologisch unbedenkliche Formulierungshilfsmittel.

**7.** Arzneimittel nach Anspruch 6 zur Behandlung von Atherosklerose.

**8.** Verwendung von neuen Arylessigsäureamiden nach Anspruch 1 bis 3 zur Herstellung von Arzneimitteln.

**9.** Verwendung nach Anspruch 8 zur Herstellung von antiatherosklerotischen Arzneimitteln.

**10.** Verwendung von neuen Arylessigsäureamiden nach Anspruch 1 bis 3 zur Verminderung der vollständigen Inhibierung der Bildung und/oder der Freisetzung von ApoB-100-assoziierten Lipoproteinen.

Europäisches Patentamt

# EUROPÄISCHER RECHERCHENBERICHT

Nummer der Anmeldung
EP 97 10 5595

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | EP 0 234 708 A (MERCK FROSST CANADA INC.) 2.September 1987<br>* Ansprüche 1,8 * | 1-4,6,8 | C07D471/04<br>A61K31/44<br>//(C07D471/04, 221:00,209:00) |
| X | US 4 906 654 A (GILLARD J.W. ET AL.) 6.März 1990<br>* Ansprüche 1,2,6-8,10 * | 1-4,6,8 | |
| X | EP 0 513 533 A (BAYER AG) 19.November 1992<br>* Ansprüche 1,2,4-9 * | 1-10 | |
| Y | EP 0 705 831 A (BAYER AG) 10.April 1996<br>* das ganze Dokument * | 1-10 | |
| P,Y | EP 0 753 517 A (BAYER AG) 15.Januar 1997<br>* das ganze Dokument * | 1-10 | |

RECHERCHIERTE SACHGEBIETE (Int.Cl.6)
C07D

Der vorliegende Recherchenbericht wurde für alle Patentansprüche erstellt

| Recherchenort | Abschlußdatum der Recherche | Prüfer |
|---|---|---|
| MÜNCHEN | 15.Juli 1997 | Hartrampf, G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)